# EUROPEAN PATENT APPLICATION

(11) **EP 4 545 075 A1**
(43) Date of publication of application: **30.04.2025**
(21) Application number: 23827190.2
(22) Date of filing: 20.06.2023
(51) Int. Cl.: A61K 31/16

(54) **CALPAIN INHIBITOR**

(30) Priority: 23.06.2022 JP 2022101432
(71) Applicant: Goryo Chemical, Inc., Sapporo-shi, Hokkaido 060-0008 (JP)
(72) Inventor: SUZUKI, Yuki, Sapporo-shi, Hokkaido 060-0008 (JP); SANO, Yusuke, Sapporo-shi, Hokkaido 060-0008 (JP)
(74) Representative: Müller-Boré & Partner Patentanwälte PartG mbB
(86) International application number: PCT/JP2023/022740
(87) International publication number: WO 2023/249007

(57) **Abstract**

The present invention provides a calpain inhibitor having an excellent calpain inhibitory ability. According to the present invention, there is provided a compound represented by the following general formula (I) or a salt thereof: 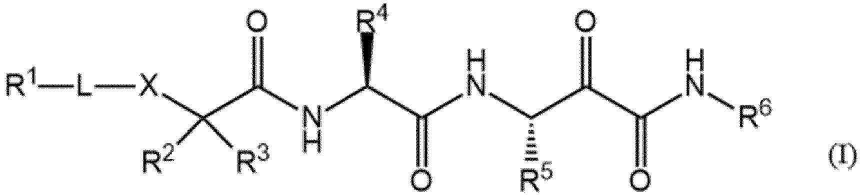 in the formula (I), R¹ represents a hydrogen atom, an alkyl group that may be substituted, or the like, or may be bonded to R² to form a ring, R² and R³ each independently represent a hydrogen atom or the like, or R² and R³ may be bonded to form a ring, provided that when R² is bonded to R¹ to form a double bond-containing ring, R³ may be absent, R⁴ represents a hydrogen atom, an alkyl group that may be substituted, or the like, R⁵ represents a hydrogen atom, an alkyl group that may be substituted, or the like, R⁶ represents a halogen, a cycloalkyl group that may be substituted, or the like, X represents O or S, when R¹ and R² are bonded to form a ring, R¹ may represent O, S, or N, and R² may represent 0 or S, and L represents an amide bond or the like.

## Description

### Technical Field

The present invention relates to a compound having a calpain inhibitory ability, and a calpain inhibitor and a pharmaceutical composition each including the compound. The present invention also relates to a method of preventing or treating a disease in which calpain activity is involved by using the compound.

### Background Art

A calpain is a cysteine protease activated in a Ca²⁺-dependent manner, and is a modulator molecule that regulates the function and structure of a matrix protein through the limited cleavage of the protein. While the calpain is involved in various life phenomena, it has been reported that the pathogenic variation of its activity or its gene is associated with various diseases, such as a neurodegenerative disease, a heart or muscle disease, an ischemic disease, a cancer, and an eye disease. Further, the calpain is essential to the infection, survival, and the like of the viruses and pathogens of some infectious diseases. For example, it has been known that the calpain is associated with a novel coronavirus infectious disease resulting from SARS coronavirus 2 or a mutant thereof, malaria, trypanosomiasis, schistosomiasis, and the like. Accordingly, it is conceivable that a calpain inhibitor that inhibits the activity of the calpain is useful in the treatment or prevention of the diseases with which the calpain is associated.

Various calpain inhibitors have heretofore been reported. In, for example, Patent Literature 1, there is a disclosure of an α-ketoamide derivative, and there is a description that the compound has a calpain inhibitory ability, and is excellent in tissue transferability and oral absorbability.

### Citation List

### Patent Literature

[PTL 1] JP 2006-76989 A

### Summary of Invention

### Technical Problem

In consideration of application to the treatment or prevention of a disease with which a calpain is associated, there still exists a demand for a calpain inhibitor having a higher calpain inhibitory ability. Accordingly, an object of the present invention is to provide a calpain inhibitor having an excellent calpain inhibitory ability.

### Solution to Problem

The inventors of the present invention have found that a calpain inhibitory ability is improved by using the α-ketoamide derivative of a peptide disclosed in Patent Literature 1 as a basic skeleton and bonding an amide group having α-carbon, which is bonded to an oxygen atom or a sulfur atom, to the N-terminal of the peptide.

The present invention relates to a compound represented by the following general formula (I) or a salt thereof: 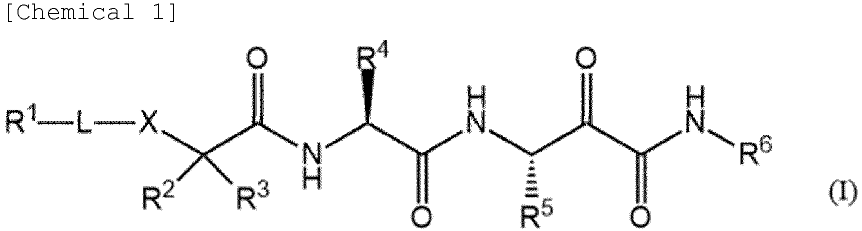 in the formula (I),
R¹ represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or may be bonded to R² to form a ring,
R² and R³ each independently represent a hydrogen atom, a hydroxyl group, an alkoxy group that may be substituted, or a linear, branched, or cyclic alkyl group that may be substituted, or R² and R³ may be bonded to form a ring, provided that when R² is bonded to R¹ to form a double bond-containing ring, R³ may be absent,
R⁴ represents a hydrogen atom, or a linear or branched alkyl group having 4 or more carbon atoms that may be substituted,
R⁵ represents a hydrogen atom, a linear or branched alkyl group that may be substituted, a linear or branched alkenyl group that may be substituted, a linear or branched alkynyl group that may be substituted, an aryl group that may be substituted, or a heterocyclic group that may be substituted,
R⁶ represents a linear, branched, or cyclic alkyl group that may be substituted, a linear or branched alkenyl group that may be substituted, a linear or branched alkynyl group that may be substituted, an aryl group that may be substituted, a heterocyclic group that may be substituted, a fused polycyclic hydrocarbon group that may be substituted, an alkoxy group that may be substituted, or a hydrogen atom,
X represents O or S,
when R¹ and R² are bonded to form a ring, R¹ may represent O, S, or N, and R² may represent O or S, and
L represents an amide bond, an ester bond, -NH-, -N-(linear, branched, or cyclic alkylene)-, or -(linear or branched alkylene-O-)ₙ- where "n" represents an integer of from 1 to 10, or L represents a single bond.

The present invention also relates to a calpain inhibitor, including the compound or the salt thereof described above.

The present invention also relates to a pharmaceutical composition for treating or preventing a calpain activity-associated disease, including the compound or the salt thereof described above.

The present invention also relates to a method of treating or preventing a calpain activity-associated disease, including administering an effective dose of the compound or the salt thereof described above to an individual in need of the treatment or prevention of the calpain activity-associated disease.

### Advantageous Effects of Invention

According to the embodiment of the present invention, the calpain inhibitory ability can be improved by using the α-ketoamide derivative of the peptide disclosed in Patent Literature 1 as a basic skeleton and bonding the amide group having α-carbon, which is bonded to an oxygen atom or a sulfur atom, to the N-terminal of the peptide. As a result, the calpain inhibitor improved in calpain inhibitory ability can be provided. Although the reason why such effects are obtained does not limit the present invention at all, and has not been elucidated, a possible reason is that the reactivity of the inhibitor with a calpain is improved.

### Brief Description of Drawings

FIG. 1 is a graph showing the results of the measurement of calpain activity.
FIG. 2 is a graph showing the results of the measurement of the calpain activity with time.
FIG. 3 is a graph showing the results of the measurement of the calpain activity.

### Description of Embodiments

Embodiments of the present invention are specifically described below, but the present invention is not limited to these embodiments. In addition, the respective embodiments may be appropriately combined as long as the effects of the present invention are obtained.

### <Definition of Terms>

Herein, the term "alkyl group" or "alkyl" moiety in any other group means a linear, branched, or cyclic saturated hydrocarbon group. Unless otherwise stated, the group is preferably a saturated hydrocarbon group having 1 to 6 carbon atoms, and examples thereof include a methyl group, an ethyl group, a n-propyl group, an isopropyl group, a n-butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a hexyl group, a 1-methylpentyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethylbutyl group, a 2-ethylbutyl group, a 1-ethyl-2-methylpropyl group, a 1,1,2-trimethylpropyl group, a cyclopropyl group, a cyclobutyl group, a cyclopentyl group, and a cyclohexyl group. The group is more preferably a C1 to C5 alkyl group, and examples thereof include a methyl group, an ethyl group, a n-propyl group, an isopropyl group, a n-butyl group, a sec-butyl group, a t-butyl group, an isobutyl group, a pentyl group, an isopentyl group, and a 2,3-dimethylpropyl group. The group is still more preferably a C1 to C3 alkyl group, and examples thereof include a methyl group, an ethyl group, a n-propyl group, and an isopropyl group. The group is most preferably a methyl group or an ethyl group. In addition, the term "alkylene group" refers to a divalent group obtained by removing one hydrogen atom from the above-mentioned alkyl group.

The term "alkoxy group" refers to a group in which the above-mentioned alkyl group is bonded via an oxygen atom ((alkyl group)-O- group), and the alkyl group moiety is as defined in the foregoing. For example, the number of carbon atoms of the alkyl group moiety in the alkoxy group may be from 1 to 6. Examples of the alkoxy group include a methoxy group, an ethoxy group, a n-propyloxy group, an isopropyloxy group, a n-butyloxy group, an isobutyloxy group, a sec-butyloxy group, a tert-butyloxy group, a pentyloxy group, an isopentyloxy group, a neopentyloxy group, a tert-pentyloxy group, a 1-methylbutyloxy group, a 2-methylbutyloxy group, a 1,2-dimethylpropyloxy group, a hexyloxy group, a 1-methylpentyloxy group, a 2-methylpentyloxy group, a 3-methylpentyloxy group, a 4-methylpentyloxy group, a 1,1-dimethylbutyloxy group, a 1,2-dimethylbutyloxy group, a 1,3-dimethylbutyloxy group, a 2,2-dimethylbutyloxy group, a 2,3-dimethylbutyloxy group, a 3,3-dimethylbutyloxy group, a 1-ethylbutyloxy group, a 2-ethylbutyloxy group, a 1-ethyl-2-methylpropyloxy group, and a 1,1,2-trimethylpropyloxy group. The C1 to C6 alkoxy group is preferably a C1 to C5 alkoxy group, more preferably a methoxy group, an ethoxy group, a n-propyloxy group, an isopropyloxy group, a n-butyloxy group, a sec-butyloxy group, a t-butyloxy group, an isobutyloxy group, a pentyloxy group, an isopentyloxy group, and a 2,3-dimethylpropyloxy group.

The term "alkenyl group" refers to a monovalent group formed by removing one hydrogen atom from any appropriate carbon atom in a linear, branched, or cyclic unsaturated hydrocarbon having one or more carbon-carbon double bonds, and the group may have, for example, 2 to 10, 2 to 6, or 2 to 4 carbon atoms. Examples of the C2 to C10 alkenyl group include a vinyl group, a propenyl group, an isopropenyl group, a 1-butenyl group, a 2-butenyl group, a 3-butenyl group, a 1-pentenyl group, a 2-pentenyl group, a 3-pentenyl group, a 4-pentenyl group, a 1-methyl-1-butenyl group, a 1-methyl-2-butenyl group, a 1-methyl-3-butenyl group, a 1-methylidenebutyl group, a 2-methyl-1-butenyl group, a 2-methyl-2-butenyl group, a 2-methyl-3-butenyl group, a 2-methylidenebutyl group, a 3-methyl-1-butenyl group, a 3-methyl-2-butenyl group, a 3-methyl-3-butenyl group, a 1-ethyl-1-propenyl group, a 1-ethyl-2-propenyl group, a 1-hexenyl group, a 2-hexenyl group, a 3-hexenyl group, a 4-hexenyl group, a 5-hexenyl group, a 1-methyl-1-pentenyl group, a 1-methyl-2-pentenyl group, a 1-methyl-3-pentenyl group, a 1-methyl-4-pentenyl group, a 1-methylidenepentyl group, a 2-methyl-1-pentenyl group, a 2-methyl-2-pentenyl group, a 2-methyl-3-pentenyl group, a 2-methyl-4-pentenyl group, a 2-methylidenepentyl group, a 3-methyl-1-pentenyl group, a 3-methyl-2-pentenyl group, a 3-methyl-3-pentenyl group, a 3-methyl-4-pentenyl group, a 3-methylidenepentyl group, a 4-methyl-1-pentenyl group, a 4-methyl-2-pentenyl group, a 4-methyl-3-pentenyl group, a 4-methyl-4-pentenyl group, a 1-heptenyl group, a 2-heptenyl group, a 3-heptenyl group, a 4-heptenyl group, a 5-heptenyl group, a 6-heptenyl group, an octenyl group, a nonenyl group, and a decenyl group.

The term "alkynyl group" refers to a monovalent group formed by removing one hydrogen atom from any appropriate carbon atom in a linear or branched unsaturated hydrocarbon having one or more carbon-carbon triple bonds, and the group may have, for example, 2 to 6 or 2 to 4 carbon atoms. Examples of the alkynyl group may include an ethynyl group, a 1-propynyl group, a 2-propynyl group, a 1-butynyl group, a 2-butynyl group, a 3-butynyl group, a pentynyl group, a hexynyl group, and a phenylethynyl group.

The term "aryl group" refers to a monovalent aromatic hydrocarbon group. The group may have, for example, 6 to 10 carbon atoms, and encompasses a phenyl group and a naphthyl group.

The term "heterocyclic group" means a monovalent group containing at least one of one or more kinds of heteroatoms selected from a nitrogen atom, an oxygen atom, and a sulfur atom, and the group is preferably from 5-membered to 14-membered. The heterocyclic group may be a monocyclic heterocyclic group or a fused heterocyclic group. The number of the heteroatoms incorporated into the 5-membered to 14-membered heterocyclic group may be, for example, from 1 to 5, from 1 to 4, from 1 to 3, 1 or 2, 2, or 1. For example, the following various combinations exist: a heterocyclic group containing 1 nitrogen atom; a heterocyclic group containing 2 nitrogen atoms; a heterocyclic group containing 3 nitrogen atoms; a heterocyclic group containing 1 oxygen atom; a heterocyclic group containing 2 oxygen atoms; a heterocyclic group containing 1 oxygen atom and 1 nitrogen atom; and a heterocyclic group containing 1 sulfur atom. The 5-membered to 14-membered heterocyclic group may be aromatic (heteroaryl group) or nonaromatic. The monocyclic heterocyclic group is preferably a 5-membered or 6-membered ring. The fused heterocyclic group is preferably an 8-membered to 10-membered ring. Examples of the 5-membered to 14-membered heterocyclic group may include piperidyl, piperazyl, morpholyl, quinuclidyl, pyrrolidyl, azetidyl, oxetyl, azetidin-2-one-yl, aziridinyl, tropanyl, furyl, tetrahydrofuryl, thienyl, pyrrolyl, pyrrolinyl, pyrrolidinyl, dioxolanyl, oxazolyl, oxazolinyl, isoxazolyl, thiazolyl, thiazolinyl, isothiazolyl, imidazolyl, imidazolinyl, imidazolidinyl, oxazolidinyl, isoxazolidinyl, thiazolidinyl, pyrazolyl, pyrazolinyl, pyrazolidinyl, oxadiazolyl, furazanyl, thiadiazolyl, 1,2,3-triazolyl, 1,2,4-triazolyl, tetrazolyl, pyranyl, pyridyl, piperidinyl, pyridazinyl, pyrimidinyl, pyrazinyl, piperazinyl, dioxanyl, oxazinyl, morpholinyl, thiomorpholinyl, thiazinyl, triazinyl, benzofuranyl, isobenzofuranyl, dihydrobenzofuranyl, dihydroisobenzofuranyl, benzothienyl, isobenzothienyl, dihydrobenzothienyl, dihydroisobenzothienyl, tetrahydrobenzothienyl, quinolyl, isoquinolyl, quinazolinyl, phthalazinyl, pteridinyl, coumaryl, chromonyl, 1,4-benzodiazepinyl, indolyl, isoindolyl, benzimidazolyl, benzofuryl, purinyl, acridinyl, phenoxazinyl, phenothiazinyl, benzoxazolyl, benzothiazolyl, indazolyl, benzimidazolyl, benzodioxolanyl, benzodioxanylchromenyl, chromanyl, isochromanyl, chromanonyl, cinnolinyl, quinoxalinyl, indolizinyl, quinolidinyl, imidazopyridyl, naphthyridinyl, dihydrobenzoxazinyl, dihydrobenzoxazolynonyl, dihydrobenzoxazinonyl, benzothioxanyl, tetrahydrofuranyl, tetrahydropyranyl, and tetrahydrothiophenyl.

Herein, the term "halogen atom" means a fluorine atom, a chlorine atom, a bromine atom, and an iodine atom, and the halogen atom is preferably a fluorine atom, a chlorine atom, and a bromine atom, more preferably a fluorine atom or a chlorine atom. Herein, the term "carboxyl group" refers to a group represented by -C(=O)-OH.

Throughout this description, examples of a substituent in the alkyl group or the alkyl moiety, the alkenyl group, or the alkynyl group may generally include a halogen atom, a hydroxyl group, an amino group, an alkylamino group, a dialkylamino group, a thiol group, an alkylthiol group, a sulfinyl group, a sulfonyl group, a thioalkyl group, an alkoxy group, a cyclic ether, a carboxyl group, an alkylcarbonyl group, an alkoxycarbonyl group, an alkoxycarbonylamino group, an alkylcarbonyloxy group, an alkylaminocarbonyl group, an alkylcarbonylamino group, a carbonylamino group, a hydrazinyl group, and a cycloalkyl group. When the alkyl group or the alkyl moiety, the alkenyl group, or the alkynyl group is substituted, the number of the substituents may be set to, for example, from 1 to 6, from 1 to 5, from 1 to 4, from 1 to 3, 1 or 2, 1, 2, or 3. Examples of a substituent with which the aryl group or the heterocyclic group may be substituted include a halogen atom and an alkoxy group. When the aryl group or the heterocyclic group is substituted, the number of the substituents may be set to, for example, from 1 to 5, 1, 2, or 3.

### <Compound represented by General Formula (I)>

In one aspect, the present invention relates to a compound represented by the following general formula (I) (hereinafter sometimes referred to as "compound (I)") or a salt thereof: 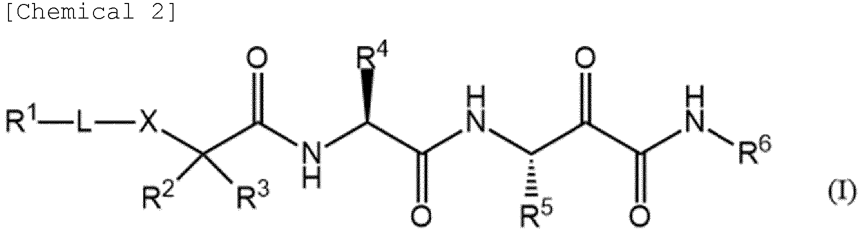 in the formula (I),
R¹ represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or may be bonded to R² to form a ring,
R² and R³ each independently represent a hydrogen atom, a hydroxyl group, an alkoxy group that may be substituted, or a linear, branched, or cyclic alkyl group that may be substituted, or R² and R³ may be bonded to form a ring, provided that when R² is bonded to R¹ to form a double bond-containing ring, R³ may be absent,
R⁴ represents a hydrogen atom, or a linear or branched alkyl group having 4 or more carbon atoms that may be substituted,
R⁵ represents a hydrogen atom, a linear or branched alkyl group that may be substituted, a linear or branched alkenyl group that may be substituted, a linear or branched alkynyl group that may be substituted, an aryl group that may be substituted, or a heterocyclic group that may be substituted,
R⁶ represents a linear, branched, or cyclic alkyl group that may be substituted, a linear or branched alkenyl group that may be substituted, a linear or branched alkynyl group that may be substituted, an aryl group that may be substituted, a heterocyclic group that may be substituted, a fused polycyclic hydrocarbon group that may be substituted, an alkoxy group that may be substituted, or a hydrogen atom,
X represents O or S,
when R¹ and R² are bonded to form a ring, R¹ may represent O, S, or N, and R² may represent O or S, and
L represents an amide bond, an ester bond, -NH-, -N-(linear, branched, or cyclic alkylene)-, or -(linear or branched alkylene-O-)ₙ- where "n" represents an integer of from 1 to 10, or L represents a single bond.

R¹ represents preferably a hydrogen atom, or a linear, branched, or cyclic alkyl group that may be substituted, more preferably a hydrogen atom, or a linear, branched, or cyclic alkyl group having 1 to 6 carbon atoms that may be substituted with one or more groups selected from a hydroxyl group, a carboxyl group, a cycloether, an alkoxy group (e.g., a methoxy group or an ethoxy group), a sulfonyl group, a halogen atom, an amino group, a methylamino group, and a dimethylamino group, still more preferably a hydrogen atom, or a linear or branched alkyl group having 1 to 4 carbon atoms (e.g., a methyl group, an ethyl group, a propyl group, an isopropyl group, or a t-butyl group) that may be substituted with one or more groups selected from a hydroxyl group, a carboxyl group, a cycloether, an alkoxy group (e.g., a methoxy group or an ethoxy group), a sulfonyl group, a halogen atom, an amino group, a methylamino group, and a dimethylamino group. Alternatively, R¹ may be preferably bonded to R² to form a 3-membered to 8-membered heterocycle containing 1 or 2 oxygen atoms and/or sulfur atoms. When R¹ and R² are bonded to form a ring, R¹ and R² may each independently represent O, S, or N. Accordingly, the heterocycle formed by the bonding of R¹ and R² may further contain a nitrogen atom. The heterocycle is as described in the foregoing for the heterocyclic group, and may be, for example, oxolanyl, oxanyl, dioxolanyl, dioxanyl, thiolanyl, thianyl, oxathiolanyl, dithiolanyl, dithianyl, oxazolidinyl, isoxazolidinyl, thiazolidinyl, or isothiazolidinyl. The heterocycle formed by the bonding of R¹ and R² may be substituted. Examples of the substituent include a halogen atom, an alkyl group (e.g., a methyl group, an ethyl group, a propyl group, or an isopropyl group), an alkoxy group (e.g., a methoxy group, an ethoxy group, a n-propyloxy group, or an isopropyloxy group), and a cyano group.

R² represents preferably a hydrogen atom, an alkyl group, or an alkoxy group, more preferably a hydrogen atom, a methyl group, or a methoxy group.

R³ represents preferably a hydrogen atom, an alkyl group, or an alkoxy group, more preferably a hydrogen atom, a methyl group, or a methoxy group.

Alternatively, R² and R³ are bonded to each other to form preferably a cycloalkyl group, more preferably a C3 to C5 cycloalkyl group.

L preferably represents -(linear or branched alkylene-O-)ₙ- where "n" represents an integer of from 1 to 10, or a single bond. The linear or branched alkylene may be, for example, an alkylene having 1 to 4 carbon atoms, preferably 2 or 3 carbon atoms, more preferably 2 carbon atoms.

X represents an oxygen atom (O) or a sulfur atom (S), preferably O.

For example, in the formula (I), a group represented by -X-L-R¹ may be a group represented by the following formula (II): 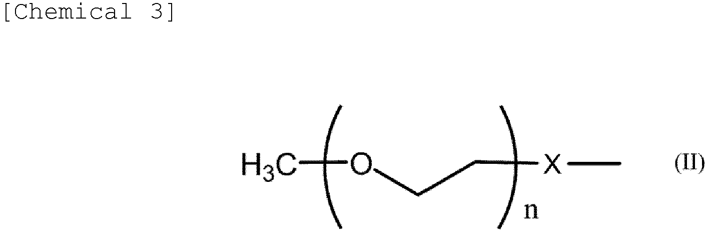 in the formula (II), "n" represents an integer of from 0 to 10.

In addition, for example, in the formula (I), the group represented by -X-L-R¹ may be a hydroxyl group, a thiol group, or a group selected from the following.

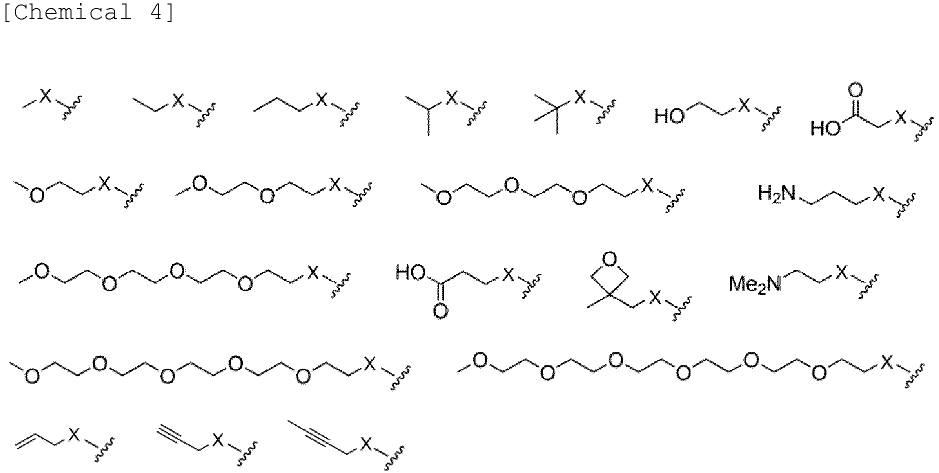

In addition, as described above, in the formula (I), R¹ and R² may be bonded to each other to form a ring. For example, a partial structure represented by 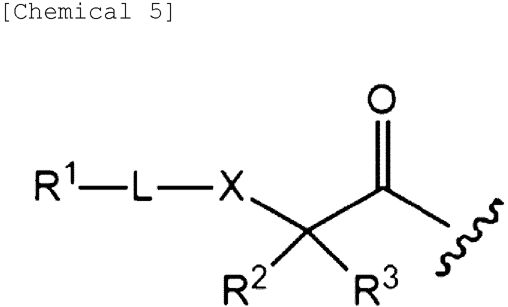 may be a structure represented by the following formula: 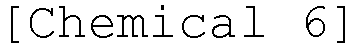 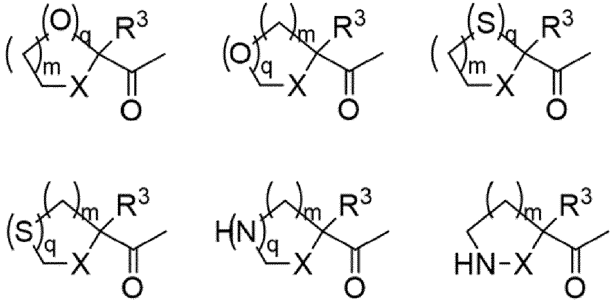 where "m" represents an integer of from 1 to 3, and "q" represents 0 or 1.

The ring formed by the bonding of R¹ and R² may be a double bond-containing ring. As described above, the double bond-containing ring may also be substituted with a substituent. The substituent is as described above. The double bond-containing ring is, for example, a 5-membered ring or a 6-membered ring. When the α-carbon of a carbonyl group in the double bond-containing ring is involved in the formation of a double bond, R³ is absent. Examples of such ring include an oxazole ring, an isoxazole ring, a furan ring, a thiophene ring, a pyran ring, a thiopyran ring, a thiazole ring, and an isothiazole ring. Accordingly, for example, in the formula (I), the partial structure represented by 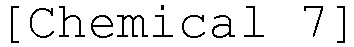 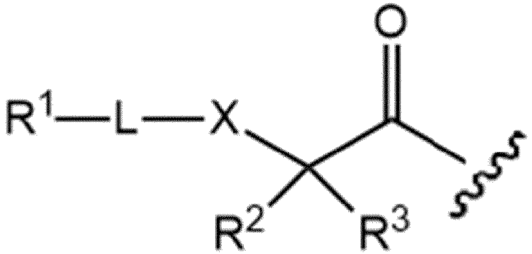 may be any one of structures represented by the following formulae: 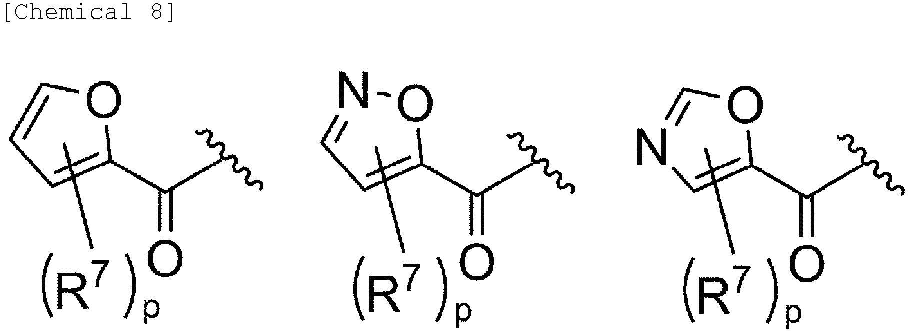 where R⁷s may be identical to or different from each other, and each represent a substituent bonded to a heterocycle, and "p" represents an integer of from 0 to 3.

Examples of the substituent represented by R⁷ include a halogen atom, an alkyl group (e.g., a methyl group, an ethyl group, a propyl group, or an isopropyl group), an alkoxy group (e.g., a methoxy group, an ethoxy group, a n-propyloxy group, or an isopropyloxy group), and a cyano group.

Examples of a substituent with which the alkyl group or the alkyl moiety, the alkenyl group, or the alkynyl group represented by R⁴ or R⁵ may be substituted include an aryl group (e.g., a phenyl group, a tolyl group, or a xylyl group), a heteroaryl group (e.g., an imidazolyl group or an indole group), an alkoxy group (e.g., a methoxy group, an ethoxy group, a n-propyloxy group, or an isopropyloxy group), a C1 to C3 thioalkyl group, an amino group, a guanidino group, an amide group, a thiol group, a carboxyl group, a hydroxyl group, and a C3 to C6 cycloalkyl group. Examples of a substituent with which the aryl group or the heterocyclic group represented by R⁵ may be substituted include a halogen atom, an alkyl group (e.g., a methyl group, an ethyl group, a propyl group, or an isopropyl group), and an alkoxy group (e.g., a methoxy group, an ethoxy group, a n-propyloxy group, or an isopropyloxy group).

In one embodiment, R⁴ and R⁵ may each independently represent a group corresponding to the side chain of an amino acid, and may each represent, for example, a group corresponding to the side chain of an amino acid selected from the group consisting of: Met; Ser; Ala; Thr; Val; Tyr; Leu; Asn; Ile; Gln; Asp; Phe; Glu; Trp; Lys; Cys; Arg; His; Gly (these amino acids are represented according to three letter codes for amino acids) ; norleucine (Nle); and methionine sulfoxide (MetO), provided that R⁴ does not represent a group corresponding to the side chain of Val.

R⁴ may preferably represent an unsubstituted C4 to C5 branched alkyl group, or a C1 to C2 alkyl group substituted with a C3 to C5 cycloalkyl group. The unsubstituted C4 to C5 branched alkyl group is preferably an isobutyl group (-CH₂CH(CH₃)₂) (the side chain of Leu) or a sec-butyl group (-CH(CH₃)CH₂CH₃) (the side chain of Ile), more preferably an isobutyl group. The C1 to C2 alkyl group substituted with the C3 to C5 cycloalkyl group is preferably a C3 to C5 cycloalkylmethyl group, more preferably a cyclopropylmethyl group or a cyclobutylmethyl group.

R⁵ represents preferably a linear or branched alkyl group that may be substituted with a phenyl group, an imidazolyl group, an amino group, a guanidino group, or a C1 to C3 thioalkyl group, more preferably a C1 to C4 linear or branched alkyl group that may be substituted with a phenyl group, an imidazolyl group, an amino group, a guanidino group, or a C1 to C3 thioalkyl group, or a phenyl group that may be substituted with a halogen atom, an alkyl group (e.g., a methyl group, an ethyl group, a propyl group, or an isopropyl group), or an alkoxy group (e.g., a methoxy group, an ethoxy group, a n-propyloxy group, or an isopropyloxy group). R⁵ may represent, for example, a benzyl group (the side chain of Phe), a phenylethyl group, a 2-(methylthio)ethyl group (the side chain of Met), a 2-(methylsulfinyl)ethyl group (the side chain of MetO), a n-butyl group (the side chain of Nle), a 1-hydroxyethyl group (the side chain of Thr), a 3-guanidinopropyl group (the side chain of Arg), a 4-aminobutyl group (the side chain of Lys), a 1H-imidazol-4-yl-methyl group (the side chain of His), a phenyl group, a n-propyl group, or an isobutyl group (the side chain of Leu), preferably a benzyl group, a phenylethyl group, a 2-(methylthio)ethyl group, a 3-guanidinopropyl group, a 2-(methylsulfinyl)ethyl group, a n-butyl group, a phenyl group, a n-propyl group, or an isobutyl group.

R⁶ represents preferably a linear, branched, or cyclic alkyl group, or an alkoxy group, more preferably a C1 to C4 linear or branched alkyl group, a C3 to C6 cycloalkyl group, or a C1 to C4 alkoxy group, still more preferably an ethyl group, a n-propyl group, an isopropyl group, a methoxy group, an ethoxy group, an isopropyloxy group, a cyclopropyloxy group, cyclopropane, or cyclobutane.

Examples of a substituent with which the alkyl group or the alkyl moiety, the alkenyl group, or the alkynyl group represented by R⁶ may be substituted include a halogen, an alkoxy group, a phenyloxy group, an alkenyl group, an alkynyl group, an amino group, an alkylamino group, a thioalkyl group, a sulfinyl group, a sulfonyl group, a carboxyl group, a nitrile group, and a hydroxyl group. Of those, a halogen, an alkoxy group, and a phenyloxy group are preferred. Examples of a substituent with which the aryl group, the heterocyclic group, or the fused polycyclic hydrocarbon group represented by R⁶ may be substituted include a halogen atom, an alkyl group (e.g., a methyl group, an ethyl group, a propyl group, an isopropyl group, or a cyclopropyl group), and an alkoxy group (e.g., a methoxy group, an ethoxy group, a n-propyloxy group, an isopropyloxy group, or a cyclopropyloxy group).

In addition to the above-mentioned alkoxy group (e.g., a methoxy group or an ethoxy group), a group obtained by further substituting the above-mentioned alkoxy group with the above-mentioned alkoxy group may also be preferably used as the alkoxy group serving as the substituent of R⁶.

The halogen serving as the substituent of R⁶ is, for example, fluorine, chlorine, or bromine. Of those, fluorine is preferred.

The fused polycyclic hydrocarbon group represented by R⁶ is, for example, indanyl, indenyl, naphthyl, anthracenyl, pentalenyl, or azulenyl. Of those, indanyl is preferred.

Specific examples of the compound in the embodiment of the present invention may include compounds represented by the following formulae: 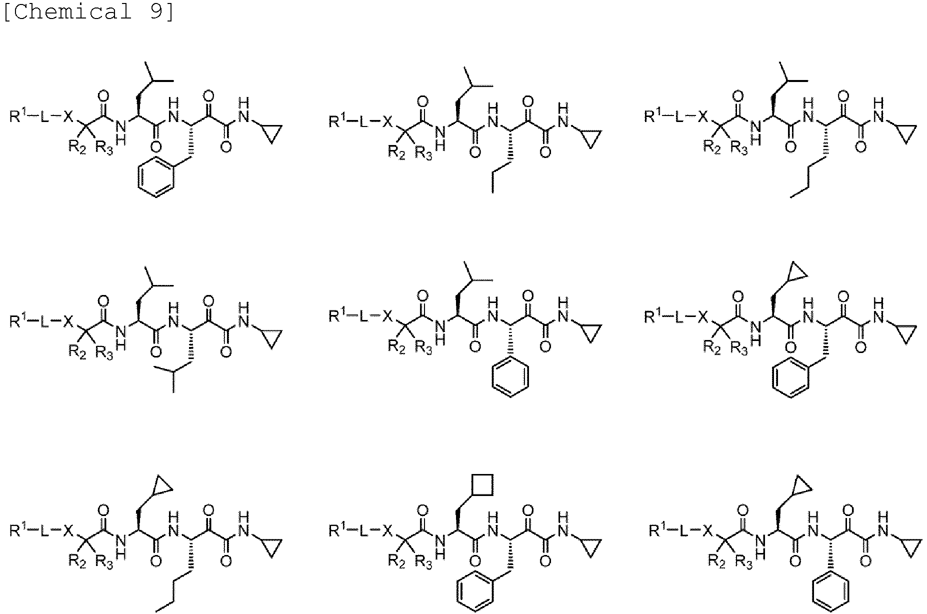 where R¹, R², and R³ are each as defined in the formula (I).

Further, specific examples of the compound in the embodiment of the present invention may also include compounds represented by the following formulae: 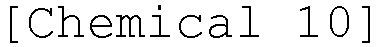 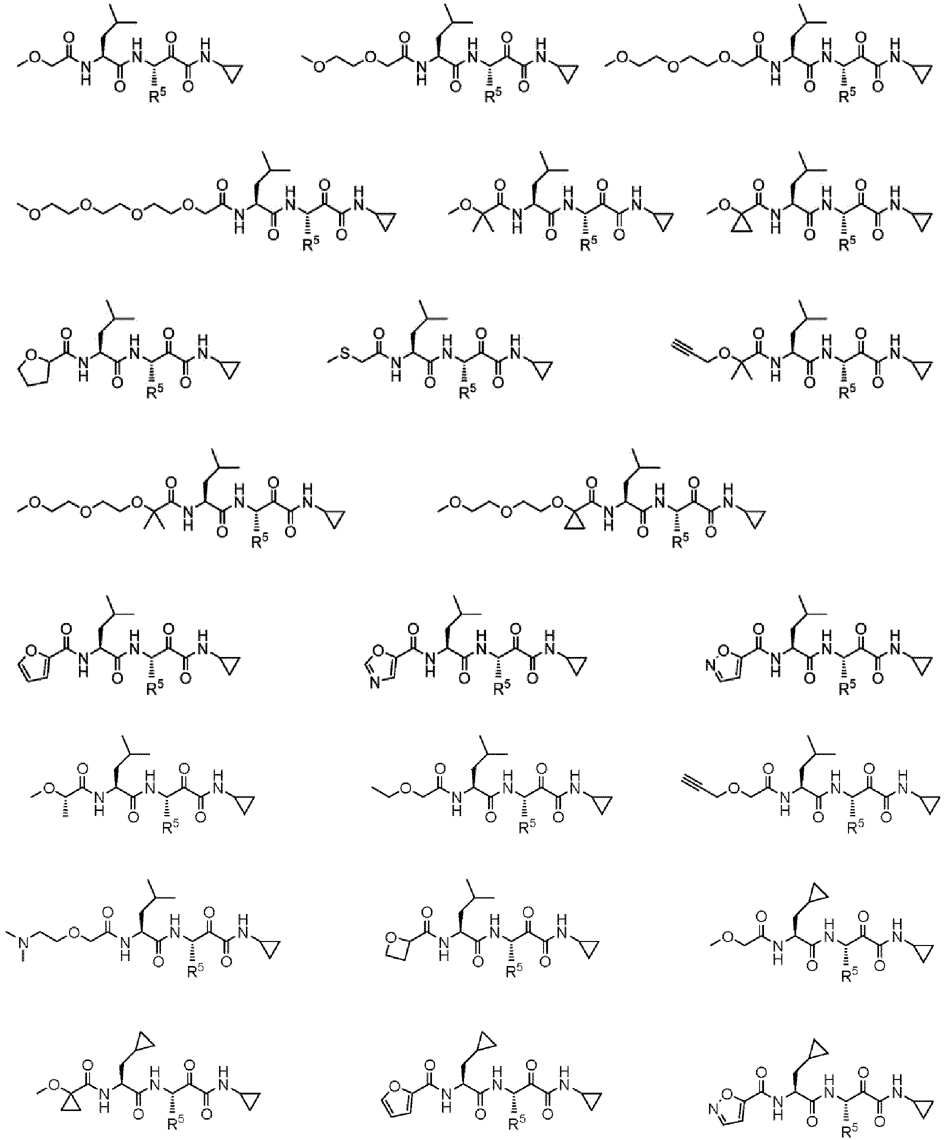 where R⁵ is as defined in the formula (I), and represents preferably a benzyl group, a phenylethyl group, a 2-(methylthio)ethyl group, a 2-(methylsulfinyl)ethyl group, a n-butyl group, a 1-hydroxyethyl group, a 3-guanidinopropyl group, a 4-aminobutyl group, a 1H-imidazol-4-yl-methyl group, a phenyl group, a n-propyl group, or an isobutyl group, more preferably a benzyl group, a 2-(methylthio)ethyl group, a phenyl group, or a n-butyl group.

The compound (I) may be synthesized by any appropriate method. The compound (I) may be synthesized with reference to, for example, a method described in Examples of the present application, or a method described in Patent Literature 1 or JP 2002-47256 A. The crude product of the compound (I) to be obtained may be further purified. The resultant crude product may be purified by, for example, reversed phase column chromatography.

Examples of the salt of the compound (I) may include a base addition salt, an acid addition salt, and an amino acid salt. Examples of the base addition salt may include: metal salts, such as a lithium salt, a sodium salt, a potassium salt, a calcium salt, and a magnesium salt; organic amine salts, such as an ammonium salt, a methylamine salt, a dimethylamine salt, a dicyclohexylamine salt, a tris(hydroxymethyl)aminomethane salt, an N,N-bis(hydroxyethyl)piperazine salt, a 2-amino-2-methyl-1-propanol salt, an ehanolamine salt, an N-methylglucamine salt, an L-glucamine salt, a triethylamine salt, a piperidine salt, and a morpholine salt; and salts with basic amino acids, such as lysine, δ-hydroxylysine, and arginine. Examples of the acid addition salt may include: mineral acid salts, such as a hydrochloric acid salt, a hydrobromic acid salt, a sulfuric acid salt, a nitric acid salt, and a phosphoric acid salt; and organic acid salts, such as a methanesulfonic acid salt, a benzenesulfonic acid salt, a para-toluenesulfonic acid salt, a citric acid salt, an oxalic acid salt, an acetic acid salt, a propionic acid salt, a tartaric acid salt, a fumaric acid salt, a maleic acid salt, a malic acid salt, a succinic acid salt, a benzoic acid salt, a mandelic acid salt, a cinnamic acid salt, a lactic acid salt, a glycolic acid salt, a glucuronic acid salt, an ascorbic acid salt, a nicotinic acid salt, and a salicylic acid salt. The amino acid salt may be, for example, a glycine salt. Of course, the salt of the compound of the present invention is not limited thereto.

The compound (I) may have one or two or more asymmetric carbon atoms in accordance with the kind of a substituent thereof, and hence a stereoisomer, such as an optical isomer or a diastereoisomer, may exist. A stereoisomer in a pure form, any appropriate mixture of stereoisomers, a racemic form, and the like are each included in the scope of the present invention. In addition, although the compound represented by the general formula (I) or the salt thereof may exist as a hydrate or a solvate, these substances are each included in the scope of the present invention. Although the kind of a solvent that forms the solvate is not particularly limited, examples thereof may include solvents, such as ethanol, acetone, and isopropanol. The reference "compound (I)" as used herein encompasses any appropriate mixture of the isomers of the compound (I) or a specific stereoisomer thereof, a salt (typically, a pharmacologically acceptable salt), hydrate, and solvate of the compound (I), and a hydrate or solvate of the salt (typically, the pharmacologically acceptable salt) of the compound (I) even when no such compound is clearly described except for the case where such compound is obviously unsuitable.

The compound (I) may have a calpain inhibitory ability that is improved as compared to that of a related-art calpain inhibitor (e.g., the calpain inhibitor disclosed in Patent Literature 1). The calpain is a gene product having a sequence region significantly homologous to the catalytic domain of human calpain-1 (µ-calpain, µCANP, µ80K, calpain I, or CDP I), and includes calpains-1 to 16. For example, calpain-1 is a heterodimer of CAPN1 and CAPNS1, and calpain-2 is a heterodimer of CAPN2 and CAPNS1. The compound (I) may have inhibitory activity on, for example, calpain-1.

In addition, the compound (I) may be excellent in water solubility. The solubility (25°C) of the compound (I) in water, physiological saline, or a buffer (e.g., a 10 mM phosphate buffer (pH 7.0)) is, for example, more than 0.1 mg/mL, and is preferably more than 0.65 mg/mL, more preferably 1.5 mg/mL or more, still more preferably 5.0 mg/mL or more. In particular, the compound (I) having an excellent solubility in water may be advantageous in terms of application to a pharmaceutical composition (e.g., an eye drop). In one embodiment, the compound (I) may have water solubility that is improved as compared to that of the related-art calpain inhibitor (e.g., the calpain inhibitor disclosed in Patent Literature 1).

### <Calpain Inhibitor or Pharmaceutical Composition>

As described above, the compound (I) may have an improved calpain inhibitory ability. Accordingly, in one aspect, the present invention relates to a calpain inhibitor or a pharmaceutical composition including the compound (I) as an active ingredient.

For example, the calpain inhibitor may be used for inhibiting the ability of a calpain. The calpain inhibitor may be used in, for example, an in vitro mode, an in vivo mode, or an ex vivo mode.

In addition, the calpain inhibitor and the pharmaceutical composition may each be used for, for example, the treatment or prevention of a calpain activity-associated disease.

The calpain activity-associated disease may be such a disease that the expression of a calpain or the activity thereof contributes to its onset or exacerbation. Examples of the calpain activity-associated disease may include: an eye disease, such as glaucoma including normal-tension glaucoma, autosomal dominant neovascular inflammatory vitreoretinopathy, retinitis pigmentosa, age-related macular degeneration, retinal neuropathy or a retinal vascular occlusive disease associated with diabetes, a retinal disease such as retinal ischemia or retinal neuropathy, or cataract; a muscle disease such as muscular dystrophy; diabetes; an inflammatory disease or an autoimmune disease, such as acute esophagitis, multiple sclerosis, an idiopathic inflammatory muscle disease, or autoimmune encephalitis; a neurological disease, such as spinal cord injury, Parkinson's disease, a neurodegenerative disease, lissencephaly, Alzheimer's disease, or spinocerebellar degeneration; a heart or blood vessel disease, such as cardiac ischemia-reperfusion injury, a chronic heart disease, an abdominal aortic aneurysm, or atherosclerosis; a cancer, such as a melanoma, breast cancer, colon cancer, kidney cancer, stomach cancer, cervical cancer, or soft tissue sarcoma; a brain tumor; an aging syndrome; progeria; a novel coronavirus infectious disease resulting from SARS coronavirus 2 or a mutant thereof; an infectious disease caused by a parasite or a pathogenic microorganism, such as a malaria infectious disease, trypanosomiasis, African sleeping sickness, schistosomiasis, leishmaniasis, or meniscocytosis; traumatic encephalopathy; Machado-Joseph disease; preeclampsia; and pulmonary fibrosis.

The subject to which the calpain inhibitor or the pharmaceutical composition is to be administered is typically a human or a mammal except the human **(e.g.,** cattle, a horse, a dog, a cat, a monkey, a pig, sheep, a rabbit, a rat, or a mouse).

The administration of the calpain inhibitor or the pharmaceutical composition may be systemic administration or local administration. The systemic administration may be performed by, for example, oral administration, intravenous injection, subcutaneous injection, or intramuscular injection. The local administration is, for example, administration to the skin, the mucous membrane, a nose, or an eye.

The dosage form of the calpain inhibitor or the pharmaceutical composition may be an oral preparation or a parenteral preparation in accordance with the administration mode thereof. Examples of the oral preparation include: liquid preparations for internal use, such as a solution, a suspension, an emulsion, and a syrup; and solid preparations for internal use, such as a tablet, a pill, a capsule, a powder, a granule, and a troche. Examples of the parenteral preparation include injections, such as a subcutaneous injection, an intravenous injection, an intramuscular injection, an intraperitoneal injection, and a vitreous injection, an eye drop, a nasal drop, and a preparation for external use **(e.g.,** an ointment such as an eye ointment) .

The calpain inhibitor or the pharmaceutical composition may further include a pharmaceutically acceptable additive in addition to the compound (I) in accordance with its dosage form or the like. The calpain inhibitor or the pharmaceutical composition may be produced by a known method such as a method described in the Japanese Pharmacopoeia.

For example, the liquid preparation for internal use may be produced by dissolving, suspending, or emulsifying the compound (I) in a diluent **(e.g.,** purified water, ethanol, or a mixed liquid thereof). The liquid preparation may further include, for example, a wetting agent, a suspending agent, an emulsifying agent, a sweetener, a flavoring agent, an aromatic, a preservative, or a buffering agent.

In addition, for example, the solid preparation for internal use is prepared by an ordinary method by mixing the compound (I) with an excipient (e.g., lactose, mannitol, glucose, microcrystalline cellulose, or starch), a binder (e.g., hydroxypropylcellulose, polyvinylpyrrolidone, or magnesium aluminometasilicate), a disintegrant (e.g., calcium cellulose glycolate), a lubricant (e.g., magnesium stearate), a stabilizing agent, a solubilizing agent (e.g., glutamic acid or aspartic acid), or the like. The preparation may be coated with a coating agent (e.g., white soft sugar, gelatin, hydroxypropylcellulose, or hydroxypropylmethylcellulose phthalate), or may be coated with two or more layers, as required.

The injection, the eye drop, or the nasal drop may be produced by dissolving or dispersing the compound (I) in a solution obtained by appropriately adding an additive, such as a tonicity-adjusting agent (e.g., sodium chloride, potassium chloride, glycerin, mannitol, sorbitol, boric acid, borax, glucose, or propylene glycol), a buffering agent (e.g., a phosphate buffer, an acetate buffer, a borate buffer, a carbonate buffer, a citrate buffer, a Tris buffer, a glutamate buffer, or an epsilon aminocaproate buffer), a preservative (e.g., methyl parahydroxybenzoate, ethyl parahydroxybenzoate, propyl parahydroxybenzoate, chlorobutanol, benzyl alcohol, benzalkonium chloride, sodium dehydroacetate, sodium edetate, boric acid, or borax), a thickener (e.g., hydroxyethylcellulose, hydroxypropylcellulose, polyvinyl alcohol, or polyethylene glycol), a stabilizer (e.g., sodium hydrogen sulfite, sodium thiosulfate, sodium edetate, sodium citrate, ascorbic acid, or dibutylhydroxytoluene), or a pH adjuster (e.g., hydrochloric acid, sodium hydroxide, phosphoric acid, or acetic acid).

The addition amount of the additive in the injection, the eye drop, or the nasal drop may be set to an appropriate amount in accordance with, for example, the kind and applications of the additive. In ordinary cases, the tonicity-adjusting agent is preferably added at from about 0.5 w/v% to about 5.0 w/v% so that the osmotic pressure of the injection, the eye drop, or the nasal drop may be from about 229 mOsm to about 343 mOsm. The addition amount of the buffering agent is preferably from about 0.01 w/v% to about 2.0 w/v%. The addition amount of the thickener is preferably from about 0.01 w/v% to about 1.0 w/v%. The addition amount of the stabilizing agent is preferably from about 0.001 w/v% to about 1.0 w/v%. The pH adjuster is added so that the pH thereof may be typically from about 3 to about 9, preferably from about 4 to about 8.

The dose of the calpain inhibitor or the pharmaceutical composition may be set to any appropriate amount in accordance with, for example, a disease of interest, a symptom, an administration subject, and an administration method. For example, when the inhibitor or the composition is administered as an internal preparation to an adult, the preparation is administered several times a day in amounts of from about 1 mg to about 200 mg (in terms of amount (i.e., titer) of the compound (I)), preferably from about 10 mg to about 100 mg (titer) each. In addition, when the inhibitor or the composition is administered as an injection to the adult, the injection is administered once a day in an amount of from about 0.1 mg to about 50 mg (titer), preferably from about 1 mg to about 30 mg (titer). In addition, when the inhibitor or the composition is locally used for an eye, an eye drop containing, for example, about 0.001 w/v% to about 1.0 w/v%, preferably about 0.01 w/v% to about 0.5 w/v% of the compound (I) is preferably dropped into the eye several times a day in amounts of from about 20 µL to about 50 µL each.

### <Prevention or Treatment Method>

In one aspect, the present invention relates to a method of treating or preventing a calpain activity-associated disease, the method including administering an effective dose of the compound (I) to an individual in need of the treatment or prevention of the calpain activity-associated disease.

The compound (I) may be administered as it is or in the form of a calpain inhibitor or a pharmaceutical composition to the individual in need of the treatment or prevention of the calpain activity-associated disease.

The calpain activity-associated disease and a method of administering the compound (I) are as described above for the calpain inhibitor or the pharmaceutical composition. In addition, the individual in need of the treatment or prevention of the calpain activity-associated disease is an individual suffering from the disease or an individual that may suffer from the disease (e.g., an individual showing calpain activity equal to or more than a reference value). As in the subject to which the calpain inhibitor or the pharmaceutical composition is to be administered, the individual is typically a human or a mammal except the human.

The effective dose of the compound (I) may be, for example, such an amount that in the case where the compound (I) is administered, a therapeutic effect or a preventive effect on the above-mentioned disease is obtained as compared to the case where the compound is not administered. A specific effective dose cannot be unconditionally set, and may be appropriately adjusted in accordance with, for example, an administration mode, an administration method, a usage purpose, and the age, body weight, and symptom of the individual.

### Examples

The present invention is described in more detail below by way of Examples. However, Examples do not limit the scope of the present invention. The entirety of literatures cited throughout the description of the present application is incorporated herein by reference.

### [Synthesis Example 1]

Compound 13 was synthesized in accordance with the following scheme.

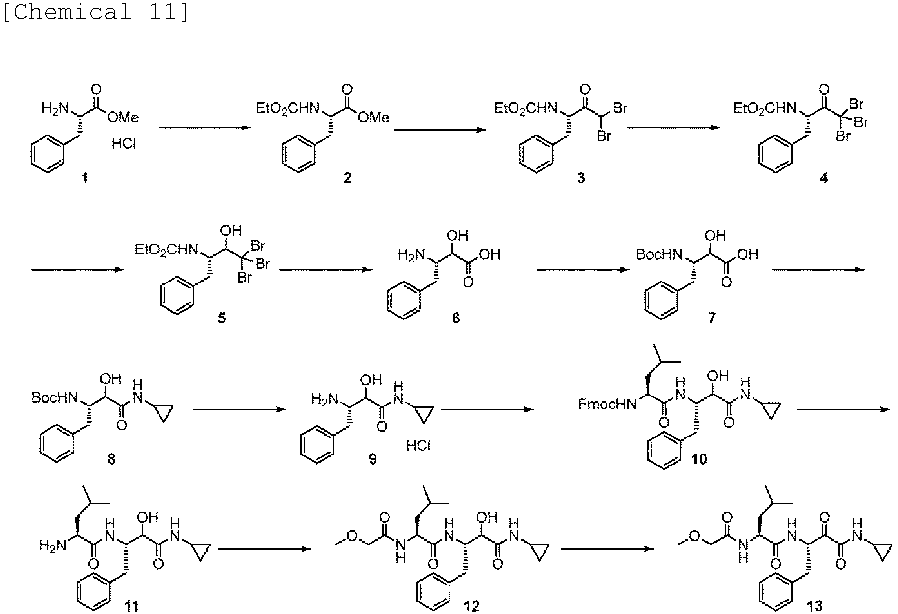

### (Synthesis of Compound 2)

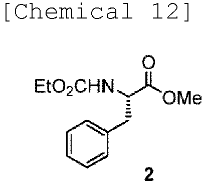

Ethyl chloroformate (5.3 mL, 55.6 mmol) was slowly added to a CH₂Cl₂ solution (92.0 mL) of Compound 1 (10.0 g, 46.4 mmol) and DIPEA (18.9 mL, 111 mmol) at 0°C, and the temperature of the mixture was gradually increased to room temperature, followed by stirring for 18 hours. Distilled water and 2 M hydrochloric acid were added to the mixture, and it was confirmed that the pH of the resultant mixture became 4. After that, the mixture was extracted with dichloromethane, and an organic phase was washed with a saturated aqueous solution of sodium hydrogen carbonate and then with a saturated saline solution. The organic phase was dried with sodium sulfate, and then the solvent was evaporated under reduced pressure. The resultant residue was purified with a silica gel column to provide Target Product 2 (11.5 g, 99%).
¹H NMR (400 MHz, CDCl₃)
δ7.28 (2H, dd, J=7.2, 2.0 Hz), 7.24 (1H, dd, J=7.2, 2.0 Hz), 7.10 (2H, dd, J=7.2, 2.0 Hz), 5.07 (1H, d), 4.63 (1H, q), 4.09 (2H, q), 4.09 (2H, q), 3.70 (3H, s), 3.09 (2H, dd), 1.21 (3H, t)

### (Synthesis of Compound 4)

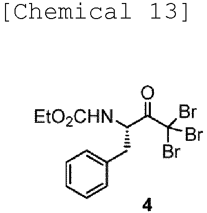

Under a nitrogen atmosphere, diisopropylamine (24.5 mL, 172 mmol) was added to n-BuMgCl (72.0 mL, 143 mmol, 2.0 M THF solution) at 40°C over 30 minutes, and the mixture was stirred at the temperature for 2 hours to provide a slurry liquid.

Under a nitrogen atmosphere, the above-mentioned slurry liquid was added to a toluene solution (20.8 mL) of Compound 2 (9.0 g, 35.8 mmol), dibromomethane (5.1 mL, 71.6 mmol), and 1,2-dimethoxyethane (10.4 mL, 100 mmol) at -10°C over 1.5 hours, and the mixture was stirred for 2 hours. Distilled water and 2 M hydrochloric acid were added to the mixture, and it was confirmed that the pH of the resultant mixture became 2. After that, the mixture was extracted with toluene, and an organic phase was washed with distilled water and then with a saturated saline solution. The organic phase was dried with sodium sulfate, and was then filtered. The resultant toluene solution was used in the next reaction.

Potassium carbonate (12.4 g, 89.5 mmol) and N-bromosuccinimide (15.9 g, 89.5 mmol) were added to the above-mentioned toluene solution at 0°C, and the mixture was stirred at the temperature for 2 hours. Distilled water was added to the mixture, and the whole was extracted with toluene, followed by the washing of an organic phase with 0.2 M hydrochloric acid and then with a saturated saline solution. The organic phase was dried with sodium sulfate, and then the solvent was evaporated under reduced pressure. Thus, Target Product 4 was obtained (15.8 g, 94%, 2 steps).
¹H NMR (400 MHz, CDCl₃)
δ7.36-7.15 (5H, m), 5.65 (1H, q), 5.13 (1H, d), 4.02 (2H, q), 3.40 (1H, dd), 3.03 (1H, dd), 1.15 (3H, t)

### (Synthesis of Compound 7)

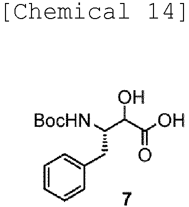

Sodium borohydride (1.27 g, 33.5 mmol) was slowly added to a MeOH solution (170 mL) of Compound 4 (15.8 g, 33.5 mmol) at 0°C, and the mixture was stirred for 2 hours. Distilled water and 2 M hydrochloric acid were added to the mixture, and it was confirmed that the pH of the resultant mixture became 4. After that, the mixture was extracted with dichloromethane, and an organic phase was washed with distilled water and then with a saturated saline solution. The organic phase was dried with sodium sulfate, and then the solvent was evaporated under reduced pressure. The resultant residue was used in the next reaction.

A 4 M aqueous solution of lithium hydroxide (83.8 mL, 335 mmol) was added to a toluene solution (80.0 mL) of the above-mentioned residue, and the mixture was stirred at 70°C for 16 hours. The mixture was cooled to room temperature, and then an organic layer and an aqueous layer were separated from each other. The aqueous layer was used in the next reaction.

THF (100 mL) and Boc₂O (14.6 g, 67.0 mmol) were added to the above-mentioned aqueous layer, and the mixture was stirred at room temperature for 18 hours. 2 M hydrochloric acid was added to the mixture, and it was confirmed that the pH of the resultant mixture became 4. After that, the mixture was extracted with ethyl acetate, and an organic phase was washed with a saturated saline solution. The organic phase was dried with sodium sulfate, and then the solvent was evaporated under reduced pressure. Thus, Target Product 7 was obtained (3.90 g, 39%, 3 steps).
¹H NMR (400 MHz, DMSO-d6)
δ7.34-7.15 (6H, m), 6.39 (1H, d), 4.00 (1H, m), 3.87 (11H, m), 2.75 (2H, m), 1.31 (9H, m)

### (Synthesis of Compound 8)

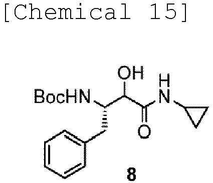

HATU (5.10 g, 13.4 mmol) was added to a DMF solution (60.0 mL) of Compound 7 (3.60 g, 12.2 mmol), DIPEA (4.30 mL, 24.4 mmol), and cyclopropylamine (1.02 mL, 14.6 mmol), and the mixture was stirred for 4 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. The resultant residue was purified with a silica gel column to provide Target Product 8 (1.17 g, 29%).
¹H NMR (400 MHz, DMSO-d6)
δ7.75 (1H, m), 7.32-7.14 (5H, m), 6.50 (0.5H, d), 6.14 (0.5H, d), 5.67 (0.5H, d), 5.50 (0.5H, d), 3.97 (1.5H, m), 3.77 (0.5H, dd), 2.78 (0.5H, m), 2.68-2.53 (2.5H, m), 1.28 (4.5H, s), 1.21 (4.5H, s), 0.58 (2H, m), 0.47 (2H, m)

### (Synthesis of Compound 9)

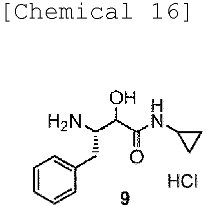

4 M hydrochloric acid (6.40 mL, 25.6 mmol, dioxane solution) was added to Compound 8 (429 mg, 1.28 mmol), and the mixture was stirred at room temperature for 1 hour. The solvent was evaporated under reduced pressure. Thus, Target Product 9 was obtained (339 mg, 97%).
HRMS (ESI⁺): calcd for [M+H]⁺, 235.1441; found, 235.1434 (-0.7 mmu)

### (Synthesis of Compound 10)

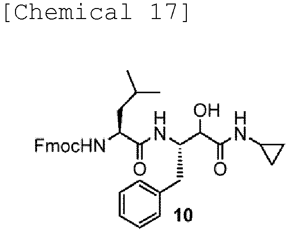

HATU (77.2 mg, 0.203 mmol) was added to a DMF solution (1.0 mL) of Compound 9 (50.0 mg, 0.185 mmol), DIPEA (0.095 mL, 0.56 mmol), and Fmoc-Leu-OH (71.8 mg, 0.203 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. The resultant residue was purified with a silica gel column to provide Target Product 10 (49.6 mg, 46%).
HRMS (ESI⁺): calcd for [M+H]⁺, 570.2962; found, 570.2955 (-0.7 mmu)

### (Synthesis of Compound 11)

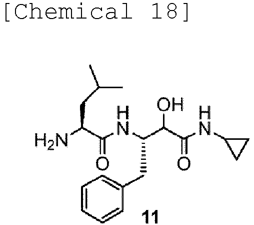

Diethylamine (1.0 mL) was added to a CH₂Cl₂ solution (4.0 mL) of Compound 10 (49.6 mg, 0.087 mmol), and the mixture was stirred at room temperature for 2 hours. The solvent was evaporated under reduced pressure, and hexane was added to the resultant residue, followed by the filtration of the resultant solid. Thus, Target Product 11 was obtained (30.3 mg, quant.). HRMS (ESI⁺): calcd for [M+H]⁺, 348.2282; found, 348.2273 (-0.9 mmu)

### (Synthesis of Compound 12)

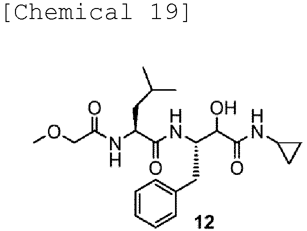

HATU (33.1 mg, 0.087 mmol) was added to a DMF solution (1.0 mL) of Compound 11 (30.3 mg, 0.087 mmol), DIPEA (0.043 mL, 0.26 mmol), and methoxyacetic acid (0.0073 mL, 0.096 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. The resultant residue was purified with a silica gel column to provide Target Product 12 (13.4 mg, 37%).
HRMS (ESI⁺): calcd for [M+H]⁺, 420.2493; found, 420.2483 (-1.0 mmu)

### (Synthesis of Compound 13)

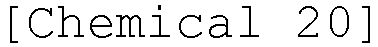

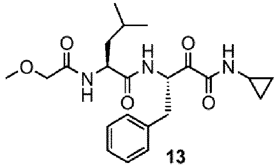

DMPI (27.1 mg, 0.064 mmol) was added to a CH₂Cl₂ solution (3.0 mL) of Compound 12 (13.4 mg, 0.032 mmol) and sodium hydrogen carbonate (43 mg, 0.26 mmol), and the mixture was stirred for 5 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 13 (6.3 mg, 47%). HRMS (ESI⁺): calcd for [M+H]⁺, 418.2336; found, 418.2331 (-0.5 mmu)
¹H NMR (400 MHz, DMSO-d6)
δ8.72 (1H, d), 8.40 (1H, d), 7.53 (1H, d), 7.30-7.08 (5H, m), 5.13 (1H, m), 4.37 (1H, m), 3.75 (2H, d), 3.23 (3H, s), 3.08 (1H, dd), 2.75 (1H, dd), 2.69 (1H, m), 1.45 (1H, m), 1.36 (2H, m), 0.82 (3H, d), 0.79 (3H, d), 0.61 (2H, m), 0.53 (2H, m)

### [Synthesis Example 2]

Compound 14 was synthesized in accordance with the following scheme.

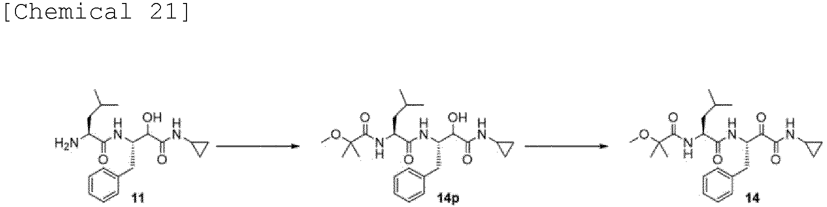

HATU (21.9 mg, 0.058 mmol) was added to a DMF solution (1.2 mL) of Compound 11 (20.0 mg, 0.058 mmol), DIPEA (0.029 mL, 0.17 mmol), and 2-methoxy-2-methylpropanoic acid (0.0071 mL, 0.063 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 14p was obtained. The compound was used in the next reaction.

DMPI (49 mg, 0.12 mmol) was added to a CH₂Cl₂ solution (1.0 mL) of Compound 14p and sodium hydrogen carbonate (19 mg, 0.23 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 14 (11.8 mg, 46%, 2 steps).
HRMS (ESI⁺): calcd for [M+H]⁺, 446.2649; found, 446.2650 (-0.1 mmu)
¹H NMR (400 MHz, DMSO-d6): 1H NMR (400 MHz, DMSO-d6):
δ8.73 (1H, d), 8.28 (1H, d), 7.46 (1H, d), 7.27-7.09 (5H, m), 5.15 (1H, m), 4.30 (1H, m), 3.06 (3H, s), 3.10 (1H, dd), 2.72 (1H, dd), 2.62 (1H, m), 1.42 (1H, m), 1.32 (2H, m), 1.19 (3H, s), 1.16 (3H, s), 0.81 (3H, d), 0.78 (3H, d), 0.61 (2H, m), 0.54 (2H, m)

### [Synthesis Example 3]

Compound 15 was synthesized in accordance with the following scheme.

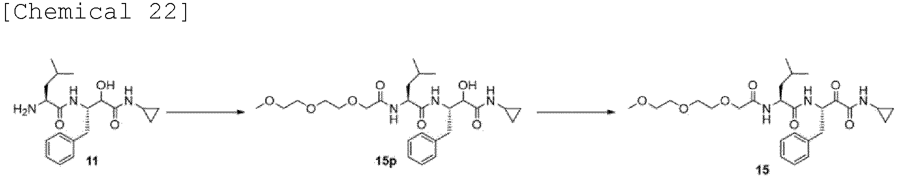

HATU (21.9 mg, 0.058 mmol) was added to a DMF solution (1.2 mL) of Compound 11 (20.0 mg, 0.058 mmol), DIPEA (0.029 mL, 0.17 mmol), and 2-[2-(2-methoxyethoxy)ethoxy] acetic acid (0.0097 mL, 0.063 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 15p was obtained. The compound was used in the next reaction.

DMPI (49 mg, 0.12 mmol) was added to a CH₂Cl₂ solution (1.0 mL) of Compound 15p and sodium hydrogen carbonate (19 mg, 0.23 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 15 (8.7 mg, 30%, 2 steps).
HRMS (ESI⁺): calcd for [M+H]⁺, 506.2861; found, 506.2856 (-0.5 mmu)
¹H NMR (400 MHz, DMSO-d6):
δ8.71 (1H, d), 8.42 (1H, d), 7.45 (1H, d), 7.30-7.07 (5H, m), 5.13 (1H, m), 4.37 (1H, m), 3.83 (2H, br-s), 3.56-3.45 (6H, m), 3.39 (2H, m), 3.19 (3H, s), 3.08 (1H, dd), 2.74 (1H, dd), 2.70 (1H, m), 1.47 (1H, m), 1.37 (2H, m), 0.83 (3H, d), 0.80 (3H, d), 0.62 (2H, m), 0.54 (2H, m)

### [Synthesis Example 4]

Compound 16 was synthesized in accordance with the following scheme.

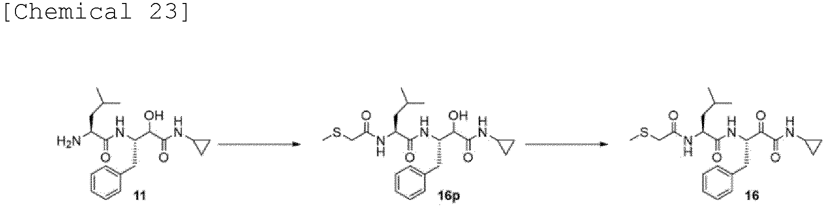

HATU (54.7 mg, 0.14 mmol) was added to a DMF solution (1.0 mL) of Compound 11 (50.0 mg, 0.14 mmol), DIPEA (0.075 mL, 0.43 mmol), and 2-(methylthio)acetic acid (0.014 mL, 0.16 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 16p was obtained.

DMPI (15 mg, 0.036 mmol) was added to a CH₂Cl₂ solution (1.0 mL) of Compound 16p (15.6 mg, 0.036 mmol) and pyridine (0.014 mL, 0.18 mmol), and the mixture was stirred for 1 hour. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 16 (6.9 mg, 44%).
HRMS (ESI⁺): calcd for [M+H]⁺, 434.2108; found, 434.2094 (-1.4 mmu)
¹H NMR (400 MHz, DMSO-d6):
δ8.70 (1H, d), 8.37 (1H, d), 7.97 (1H, d), 7.29-7.08 (5H, m), 5.15 (1H, m), 4.33 (1H, m), 3.07 (1H, dd), 3.02 (2H, d), 2.76 (1H, dd), 2.68 (1H, m), 1.99 (3H, s), 1.52 (1H, m), 1.34 (2H, m), 0.83 (3H, d), 0.80 (3H, d), 0.61 (2H, m), 0.53 (2H, m)

### [Synthesis Example 5]

Compound 17 was synthesized in accordance with the following scheme.

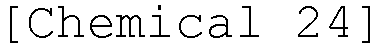

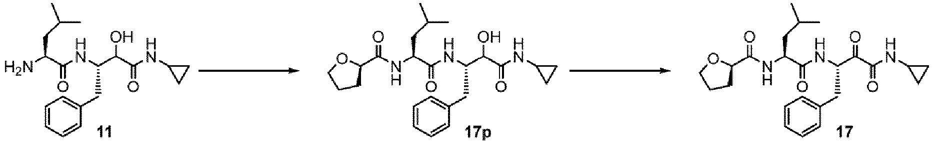

HATU (22.0 mg, 0.058 mmol) was added to a DMF solution (0.5 mL) of Compound 11 (20.0 mg, 0.058 mmol), DIPEA (0.029 mL, 0.17 mmol), and (2R)-tetrahydro-2-furancarboxylic acid (0.006 mL, 0.063 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 17p was obtained. The compound was used in the next reaction.

DMPI (49 mg, 0.12 mmol) was added to a CH₂Cl₂ solution (0.5 mL) of Compound 17p and sodium hydrogen carbonate (19 mg, 0.23 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 17 (5.4 mg, 21%, 2 steps).

HRMS (ESI⁺): calcd for [M+H]⁺, 444.2493; found, 444.2473 (-2.0 mmu)

¹H NMR (400 MHz, DMSO-d6):
δ8.78 (1H, d), 8.38 (1H, d), 7.48 (1H, d), 7.27-7.07 (5H, m), 5.13 (1H, m), 4.30 (1H, m), 3.80 (1H, m), 3.69 (1H, m), 3.10 (1H, m), 2.73 (1H, dd), 2.66 (1H, m), 2.01 (1H, m), 1.71 (4H, m), 1.40 (1H, m), 1.30 (2H, m), 0.82 (3H, d), 0.78 (3H, d), 0.62 (2H, m), 0.54 (2H, m)

### [Synthesis Example 6]

Compound 18 was synthesized in accordance with the following scheme.

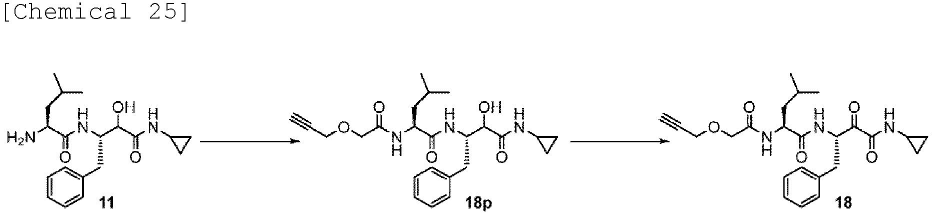

HATU (22.0 mg, 0.058 mmol) was added to a DMF solution (0.5 mL) of Compound 11 (20.0 mg, 0.058 mmol), DIPEA (0.029 mL, 0.17 mmol), and 2-(2-propyn-1-yloxy)acetic acid (0.0072 mL, 0.063 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 18p was obtained (7.2 mg, 28%).

DMPI (13.7 mg, 0.032 mmol) was added to a CH₂Cl₂ solution (1.0 mL) of Compound 18p (7.2 mg, 0.016 mmol) and sodium hydrogen carbonate (54 mg, 0.065 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 18 (5.1 mg, 71%).
HRMS (ESI⁺): calcd for [M+H]⁺, 442.2336; found, 442.2313 (-2.3 mmu)
¹H NMR (400 MHz, DMSO-d6):
δ8.70 (1H, d), 8.40 (1H, d), 7.59 (1H, d), 7.39-7.10 (5H, m), 5.12 (1H, m), 4.36 (1H, m), 4.16 (2H, d), 3.87 (2H, s), 3.50 (1H, m), 3.08 (1H, dd), 2.75 (1H, dd), 2.68 (1H, m), 1.46 (1H, m), 1.36 (2H, m), 0.82 (3H, d), 0.79 (3H, d), 0.61 (2H, m), 0.53 (2H, m)

### [Synthesis Example 7]

Compound 19 was synthesized as a comparative compound in accordance with the following scheme.

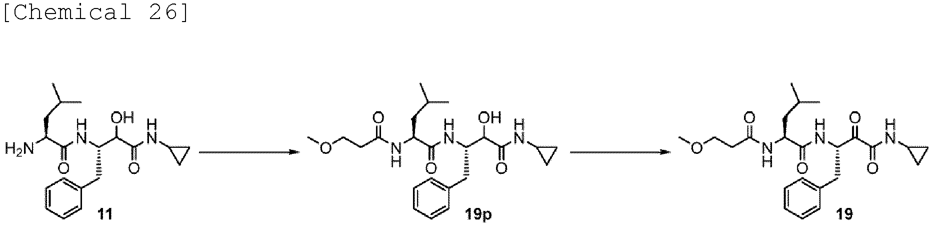

HATU (22.0 mg, 0.058 mmol) was added to a DMF solution (0.5 mL) of Compound 11 (20.0 mg, 0.058 mmol), DIPEA (0.029 mL, 0.17 mmol), and 3-methoxypropanoic acid (6.6 mg, 0.063 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 19p was obtained (7.7 mg, 31%).

DMPI (15 mg, 0.035 mmol) was added to a CH₂Cl₂ solution (1.0 mL) of Compound 19p (7.7 mg, 0.018 mmol) and sodium hydrogen carbonate (6.0 mg, 0.071 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 19 (3.4 mg, 44%).
HRMS (ESI⁺): calcd for [M+H]⁺, 432.2493; found, 432.2471 (-2.2 mmu)
¹H NMR (400 MHz, DMSO-d6):
δ8.69 (1H, d), 8.25 (1H, d), 7.87 (1H, d), 7.28-7.09 (5H, m), 5.12 (1H, m), 4.30 (1H, m), 3.43 (2H, m), 3.14 (3H, s), 3.07 (1H, dd), 2.76 (1H, dd), 2.67 (1H, m), 2.27 (2H, m), 1.51 (1H, m), 1.30 (2H, m), 0.82 (3H, d), 0.77 (3H, d), 0.61 (2H, m), 0.53 (2H, m)

### [Synthesis Example 8]

Compound 27 was synthesized in accordance with the following scheme.

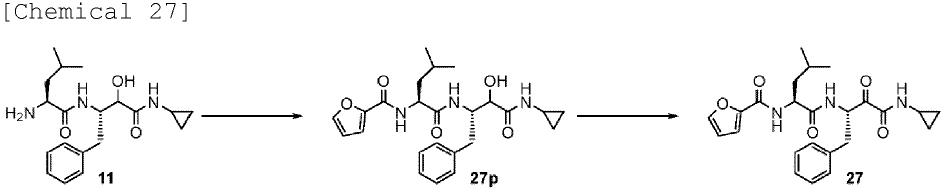

HATU (30.0 mg, 0.079 mmol) was added to a DMF solution (1.0 mL) of Compound 11 (25.0 mg, 0.072 mmol), DIPEA (0.037 mL, 0.22 mmol), and 2-furoic acid (8.8 mg, 0.063 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 27p was obtained.

DMPI (45.0 mg, 0.11 mmol) was added to a CH₂Cl₂ solution (1.0 mL) of Compound 27p and sodium hydrogen carbonate (18.0 mg, 0.21 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 27 (13.0 mg, 41%, 2 steps).
HRMS (ESI⁺): calcd for [M+H]⁺, 440.2180; found, 440.2156 (-2.4 mmu)
¹H NMR (400 MHz, CDCl₃):
δ7.45 (1H, m), 7.14-7.09 (4H, m), 7.07-7.01 (2H, m), 6.90 (1H, d), 6.69 (1H, d), 6.53 (1H, d), 6.52 (1H, d), 5.54 (1H, m), 4.57 (1H, m), 3.33 (1H, dd), 3.02 (1H, dd), 2.79 (1H, m), 1.65 (1H, m), 1.55 (2H, m), 0.91 (3H, d), 0.89 (3H, d), 0.86 (2H, m), 0.60 (2H, m)

### [Synthesis Example 9]

Compound 30 was synthesized in accordance with the following scheme.

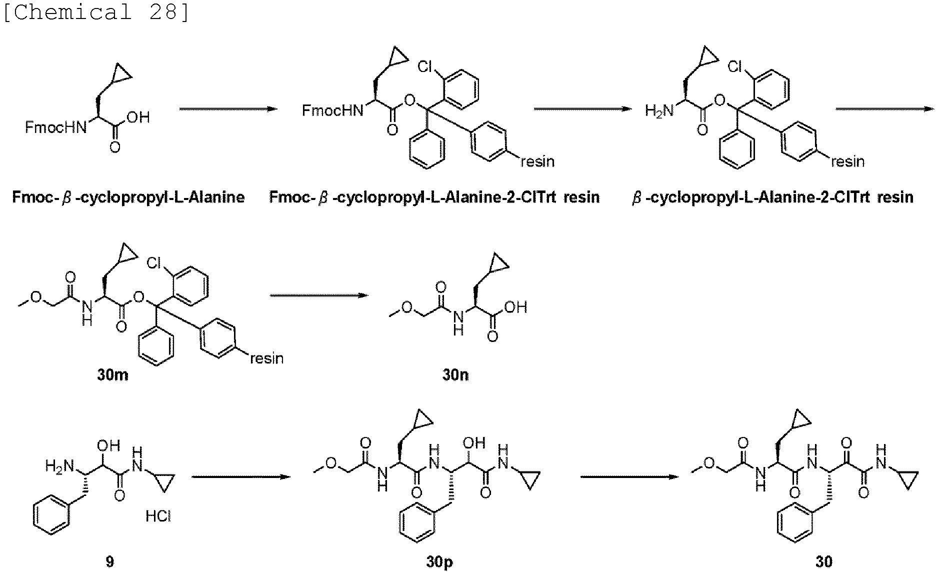

### (Synthesis of Compound 30n)

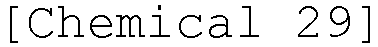

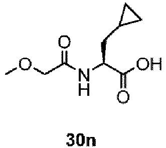

Fmoc-β-cyclopropyl-L-alanine (200 mg, 0.57 mmol), DIPEA (0.30 mL, 1.72 mmol), a 2-chlorotrityl chloride resin (244 mg, 0.38 mmol), and CH₂Cl₂ (4.0 mL) were loaded into a plastic vessel, and were mixed well by shaking for 17 hours. The reaction solution was filtered, and the filtered product was washed with CH₂Cl₂ (5.0 mL).

The resultant filtered product was transferred to a plastic vessel. A solution (5.0 mL) containing piperidine and DMF at 1:4 was loaded thereinto, and the contents were mixed well by shaking for 1 hour. The reaction solution was filtered, and the filtered product was washed with DMF (5.0 mL) and then with CH₂Cl₂ (5.0 mL).

The resultant filtered product was transferred to a plastic vessel. DIPEA (0.27 mL, 1.55 mmol), methoxyacetic acid (0.058 mL, 0.76 mmol), a DMF solution (5.0 mL), and HATU (0.29 g, 0.76 mmol) were loaded thereinto, and the contents were mixed well by shaking for 3 hours. The reaction solution was filtered, and the filtered product was washed with DMF (5.0 mL) and then with CH₂Cl₂ (5.0 mL).

The resultant filtered product was transferred to a plastic vessel. TFA (4.0 mL) was loaded thereinto, and the mixture was left at rest for 2 hours. After that, the reaction solution was filtered, and the filtered product was washed with TFA (4.0 mL). The resultant filtrate was concentrated under reduced pressure to provide Target Product 30n (76 mg, 99%, 4 steps).

### (Synthesis of Compound 30)

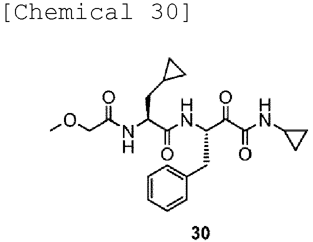

HATU (56 mg, 0.15 mmol) was added to a DMF solution (1.5 mL) of Compound 9 (40 mg, 0.15 mmol), DIPEA (0.075 mL, 0.44 mmol), and Compound 30n (30 mg, 0.15 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 30p was obtained (18 mg, 29%).

DMPI (28 mg, 0.066 mmol) was added to a CH₂Cl₂ solution (1.0 mL) of Compound 30p (18 mg, 0.043 mmol) and sodium hydrogen carbonate (11 mg, 0.13 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 30 (4.8 mg, 27%).
HRMS (ESI⁺): calcd for [M+H]⁺, 416.2180; found, 416.2153 (-2.7 mmu)
¹H NMR (400 MHz, DMSO-d6):
δ8.71 (1H, d), 8.39 (1H, d), 7.52 (1H, d), 7.30-7.09 (5H, m), 5.16 (1H, m), 4.38 (1H, m), 3.76 (2H, d), 3.25 (3H, s), 3.08 (1H, dd), 2.75 (1H, dd), 2.65 (1H, m), 1.43 (2H, m), 0.66-0.47 (5H, m), 0.30 (2H, m), 0.00 (2H, m)

### [Synthesis Example 10]

Compound 35 was synthesized in accordance with the following scheme.

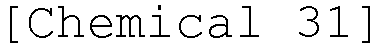

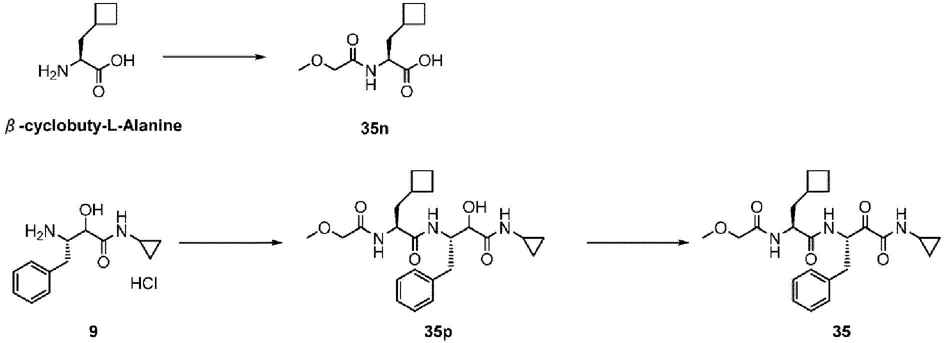

### (Synthesis of Compound 35n)

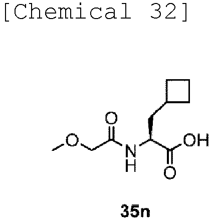

A 2 M aqueous solution (0.70 mL) of sodium hydroxide was added to β-cyclobutyl-L-alanine (0.10 g, 0.70 mmol) to dissolve the compound. After that, methoxyacetic acid (0.083 mL, 0.90 mmol) was added to the solution, and the mixture was stirred at room temperature for 5 hours. 2 M hydrochloric acid was added to the mixture, and it was confirmed that the pH of the resultant mixture became 4. After that, the mixture was extracted with ethyl acetate, and an organic phase was washed with a saturated saline solution. The organic phase was dried with sodium sulfate, and then the solvent was evaporated under reduced pressure. MTBE (1.0 mL) and hexane (5.0 mL) were added to the resultant residue, and the resultant solid was filtered to provide Compound 35n (12 mg, 8%).

### (Synthesis of Compound 35)

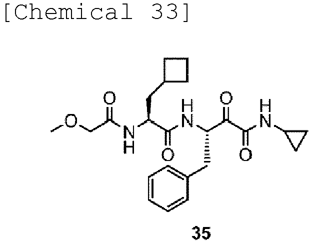

HATU (21 mg, 0.056 mmol) was added to a DMF solution (1.0 mL) of Compound 9 (20 mg, 0.073 mmol), DIPEA (0.029 mL, 0.17 mmol), and Compound 35n (12 mg, 0.056 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 35p was obtained (12 mg, 49%).

DMPI (18 mg, 0.043 mmol) was added to a CH₂Cl₂ solution (1.0 mL) of Compound 35p (12 mg, 0.028 mmol) and sodium hydrogen carbonate (7.1 mg, 0.086 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 35 (3.0 mg, 25%).
HRMS (ESI⁺): calcd for [M+H]⁺, 430.2336; found, 430.2336 (0.0 mmu)
¹H NMR (400 MHz, DMSO-d6) :
δ8.72 (1H, d), 8.38 (1H, d), 7.42 (1H, d), 7.28-7.12 (5H, m), 5.13 (1H, m), 4.24 (1H, m), 3.74 (2H, d), 3.24 (3H, s), 3.08 (1H, dd), 2. 74 (1H, dd), 2.70 (1H, m), 2.15 (1H, m), 1.90 (1H, m), 1.90 (1H, m), 1.75-1.48 (6H, m), 0.62 (2H, m)), 0.54 (2H, m)

### [Synthesis Example 11]

Compound 36 was synthesized in accordance with the following scheme.

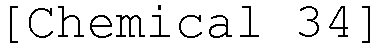

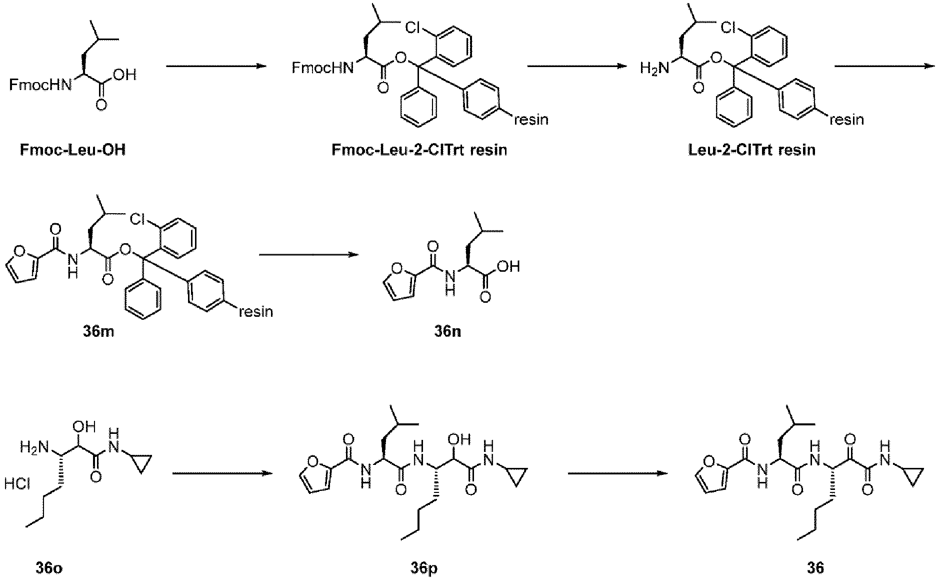

### (Synthesis of Compound 36n)

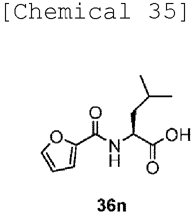

Fmoc-Leu-OH (2.5 g, 7.1 mmol), DIPEA (3.8 mL, 22 mmol), a 2-chlorotrityl chloride resin (3.1 g, 4.8 mmol), and CH₂Cl₂ (16 mL) were loaded into a plastic vessel, and were mixed well by shaking for 20 hours. The reaction solution was filtered, and the filtered product was washed with CH₂Cl₂ (15 mL).

A part (0.19 g, 0.20 mmol) of the resultant filtered product was transferred to a plastic vessel. A solution (0.4 mL) containing piperidine and DMF at 1:4 was loaded thereinto, and the contents were mixed well by shaking for 1 hour. The reaction solution was filtered, and the filtered product was washed with DMF (1.0 mL) and then with CH₂Cl₂ (1.0 mL).

The resultant filtered product was transferred to a plastic vessel. DIPEA (0.14 mL, 0.82 mmol), 2-furoic acid (45 mg, 0.40 mmol), a DMF solution (2.0 mL), and HATU (0.15 g, 0.40 mmol) were loaded thereinto, and the contents were mixed well by shaking for 3 hours. The reaction solution was filtered, and the filtered product was washed with DMF (3.0 mL) and then with CH₂Cl₂ (3.0 mL).

The resultant filtered product was transferred to a plastic vessel. TFA (2.0 mL) was loaded thereinto, and the mixture was left at rest for 2 hours. After that, the reaction solution was filtered, and the filtered product was washed with TFA (2.0 mL). The resultant filtrate was concentrated under reduced pressure to provide Target Product 36n (45 mg, quant., 3 steps).

### (Synthesis of Compound 36)

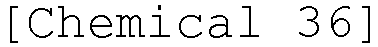

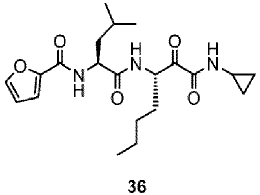

HATU (32 mg, 0.084 mmol) was added to a DMF solution (1.0 mL) of Compound 36o (20 mg, 0.084 mmol), which is commercially available (ChemScene LLC), DIPEA (0.043 mL, 0.25 mmol), and Compound 36n (19 mg, 0.084 mmol), and the mixture was stirred for 2 hours. 0.2 M hydrochloric acid was added to the mixture, and it was confirmed that the pH of the resultant mixture became 4. After that, the mixture was extracted with MTBE, and an organic phase was washed with a saturated saline solution. The organic phase was dried with sodium sulfate, and then the solvent was evaporated under reduced pressure. Thus, Compound 36p was obtained (34 mg, 99%).

DMPI (53 mg, 0.13 mmol) was added to a CH₂Cl₂ solution (1.0 mL) of Compound 36p (34 mg, 0.083 mmol) and sodium hydrogen carbonate (21 mg, 0.25 mmol), and the mixture was stirred for 2 hours. A saturated aqueous solution of sodium hydrogen carbonate and a saturated aqueous solution of sodium thiosulfate were added to the mixture, and the whole was extracted with CH₂Cl₂, followed by the washing of an organic phase with a saturated saline solution. The organic phase was dried with sodium sulfate, and then the solvent was evaporated under reduced pressure. The resultant residue was purified with a silica gel column to provide Target Product 36 (31 mg, 92%).
HRMS (ESI⁺): calcd for [M+H]⁺, 406.2336; found, 406.2304 (-3.2 mmu)
¹H NMR (400 MHz, DMSO-d6):
δ8.68 (1H, d), 8.29 (1H, d), 8.15 (1H, d), 7.80 (1H, s), 7.15 (1H, d), 6.58 (1H, dd), 4.90 (1H, m), 4.50 (1H, m), 2.69 (1H, m), 1.70 (1H, m), 1.57 (2H, m), 1.45 (2H, m), 1.37-1.14 (4H, m), 0.92-0.75 (9H, m), 0.60 (2H, m), 0.52 (2H, m)

Compounds 13 to 18, 27, 30, 35, and 36 synthesized in Synthesis Examples 1 to 11 described above, Compounds 20, 22, 25, 26, 28, 29, 31 to 34, and 37 to 43 synthesized in accordance with these synthesis examples, and Compounds 21, 23, 24, and 44 that can be synthesized in accordance with these synthesis examples are shown in Tables 1A to 1D.

**[Table 1A]**

| Compound No. | Compound | 1H NMR (400MHz) | HRMS(ESI⁺) |
|---|---|---|---|
| 13 | 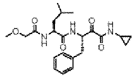 | DMSO-d6 | calcd for [M+H]⁺, 41 8. 2336 |
| | | 8. 72 (1H, d), 8. 40 (1H, d), 7. 53 (1H, d), 7. 30-7. 08 (5H, m), 5. 13 (1H, m), 4. 37 (1H, m), 3. 75 (2H, d), 3. 23 (3H, s), 3. 08 (1H, dd), 2. 75 (1H, dd), 2. 69 (1H, m), 1. 45 (1H, m), 1. 36 (2H, m), 0. 82 (3H, d), 0. 79 (3H, d), 0. 61 (2H, m), 0. 53 (2H, m) | |
| | | | found, 418. 2331 (-0.5 mmu) |
| 14 | 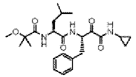 | DMSO-d6 | calcd for [M+H]⁺, 44 6.2649 |
| | | δ8. 73 (1H, d), 8. 28 (1H, d), 7. 46 (1H, d), 7. 27-7. 09 (5 H, m), 5. 15 (1H, m), 4. 30 (1H, m), 3. 06 (3H, s), 3. 10 (1H, dd), 2. 72 (1H, dd), 2. 62 (1H, m), 1. 42 (1H, m), 1. 32 (2H, m), 1. 19 (3H, s), 1. 16 (3H, s), 0. 81 (3H, d), 0.78 (3H, d), 0. 61 (2H, m), 0. 54 (2H, m) | |
| | | | found, 446. 2650 (-0.1mmu) |
| 15 | 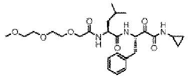 | DMSO-d6 | calcd for [M+H]⁺, 50 6.2861 |
| | | δ8. 71 (1H, d), 8. 42 (1H, d), 7. 45 (1H, d), 7. 30-7. 07 (5H, m), 5. 13 (1H, m), 4. 37 (1H, m), 3. 83 (2H, br-s), 3. 56-3. 45 (6H, m), 3. 39 (2H, m), 3. 19 (3H, s), 3. 08 (1H, dd), 2. 74 (1H, dd), 2. 70 (1H, m), 1. 47 (1H, m), 1. 37 (2H, m), 0. 83 (3H, d), 0. 80 (3H, d), 0. 62 (2H, m), 0. 54 (2H, m) | |
| | | | found, 506. 2856 (-0.5mmu) |
| 16 | 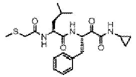 | DMSO-d6 | calcd for [M+H]⁺, 43 4.2108 |
| | | δ8. 70 (1H, d), 8. 37 (1H, d), 7. 97 (1H, d), 7. 29-7. 08 (5H, m), 5. 15 (1H, m), 4. 33 (1H, m), 3. 07 (1H, dd), 3. 02 (2H, d), 2. 76 (1H, dd), 2. 68 (1H, m), 1. 99 (3H, s), 1. 52 (1H, m), 1. 34 (2H, m), 0. 83 (3H, d), 0. 80 (3H, d), 0. 61 (2H, M, m), 0. 53 (2H, m) | |
| | | | found, 434. 2094 (-1.4mmu) |
| 17 | 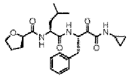 | DMSO-d6 | calcd for [M+H]+, 44 4.2493 |
| | | δ8. 78 (1H, d), 8. 38 (1H, d), 7. 48 (1H, d), 7. 27-7. 07 (5H, m), 5. 13 (1H, m), 4. 30 (1H, m), 3. 80 (1H, m), 3. 69 (1H, m), 3. 10 (1H, m), 2. 73 (1H, dd), 2. 66 (1H, m), 2. 01 (1H, m), 1. 71 (4H, m), 1. 40 (1H, m), 1. 30 (2H, m), 0. 82 (3H, d), 0. 78 (3H, d), 0. 62 (2H, m), 0. 54 (2H, m) | |
| | | | found, 444. 2473 (-2.0mmu) |
| 18 | 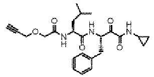 | DMSO-d6 | calcd for [M+H]⁺, 44 2. 2336 |
| | | δ8. 70 (1H, d), 8. 40 (1H, d), 7. 59 (1H, d), 7. 39-7. 10 (5H, m), 5. 12 (1H, m), 4. 36 (1H, m), 4. 16 (2H, d), 3. 87 (2H, s), 3. 50 (1H, m), 3. 08 (1H, dd), 2. 75 (1H, dd), 2. 68 (1H, m), 1. 46 (1H, m), 1. 36 (2H, m), 0. 82 (3H, d), 0. 79 (3H, d), 0. 61 (2H, m), 0. 53 (2H, m) | |
| | | | found, 442. 2313 (-2.3mmu) |
| 20 | 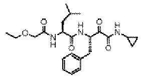 | DMSO-d6 | calcd for [M+H]⁺, 43 2. 2493 |
| | | δ8. 71 (1H, d), 8. 42 (1H, d), 7. 43 (1H, d), 7. 28-7. 12 (5H, m), 5.13 (1H, m), 4. 38 (1H, m), 3. 77 (2H, d), 3. 41 (2H, q), 3. 08 (1H, dd), 2. 75 (1H, dd), 2. 66 (1H, m), 1. 46 (1H, m), 1. 37 (2H, m), 1. 08 (3H, t), 0. 81 (3H, d), 0. 80 (3H, d), 0. 61 (2H, m), 0. 53 (2H, m) | |
| | | | found, 432. 2467 (-2. 6mmu) |

**[Table 1B]**

| | | | |
|---|---|---|---|
| 21 | 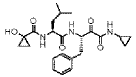 | | |
| 22 | 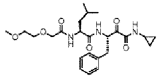 | | calcd for [M + H]⁺, 46 2. 2599 |
| | | | found, 462. 2557 (-4. 2mmu) |
| 23 | 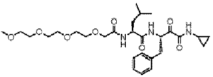 | | |
| 24 | 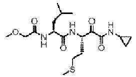 | | |
| 25 | 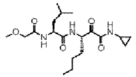 | DMSO-d6 | calcd for [M+M]⁺, 38 4.2193 |
| | | δ8. 68 (1H, d), 8. 30 (1H, d), 7. 56 (1H, d), 4. 89 (1H, m), 4. 41 (1H, m), 3. 77 (2 H, d), 3. 26 (3H, s), 2. 70 (1H, m), 1. 70 (1H, m), 1. 53 (2H, m), 1. 41 (2H, m), 1. 35-1. 14 (4H, m), 0. 89-0. 77 (9H, m), 0. 61 (2H, m), 0. 53 (2H, m) | found, 384.2469 (-2. 4mmu) |
| 26 | 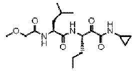 | DMSO-d6 | calcd for [M+H]⁺, 37 0.2336 |
| | | δ8. 68 (1H, d), 8. 30 (1H, d), 7. 55 (1H, d), 4. 90 (1H, m), 4. 41 (1H, m), 3. 78 (2 H, d), 3. 26 (3H, s), 2. 70 (1H, m), 1. 66 (1H, m), 1. 43 (2H, m), 1. 37-1. 16 (4 H, m), 0. 88-0. 78 (9H, m), 0. 60 (2H, m) . 0. 53 (2H, m) | |
| | | | found, 370.2332 (-0. 4mmu) |
| 27 | 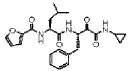 | CDCl₃ | calcd for [M+H]⁺, 44 0.2180 |
| | | δ7. 45 (1H, m), 7. 14-7. 09 (4H, m), 7. 07-7. 01 (2H, m), 6. 90 (1H, d), 6. 69 (1 H, d), 6. 53 (1H, d), 6. 52 (1H, d), 5. 54 (1H, m), 4. 57 (1H, m), 3. 33 (1H, dd), 3. 02 (1H, dd), 2. 79 (1H, m), 1. 65 (1H, m), 1. 55 (2H, m), 0. 91 (3H, d), 0. 89 (3H, d), 0. 86 (2H, m), 0. 60 (2H, m) | |
| | | | found, 440.2156 (-2. 4mmu) |
| 28 | 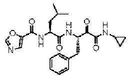 | DMSO-d6 | calcd for [M+H]⁺, 44 1.2132 |
| | | δ8. 69 (1H, d), 8. 51 (1H, s), 8. 50 (1H, d), 8. 41 (1H, d), 7. 81 (1H, s), 7. 25-7. 09 (5H, m), 5. 12 (1H, m), 4. 47 (1H, m), 3.07 (1H, dd), 2. 78 (1H, dd), 2. 64 (1H, m), 1. 52 (2H, m), 1. 41 (1H, m), 0. 84 (3H, d), 0. 80 (3H, d), 0. 60 (2H, m), 0. 52 (2H, m) | |
| | | | found, 441.2103 (-2. 9mmu) |

**[Table 1C]**

| | | | |
|---|---|---|---|
| 29 | 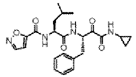 | DMSO-d6 | calcd for [M+H]⁺, 44 1.2132 |
| | | δ8. 84 (1H, d), 8. 70 (1H, d), 8. 68 (1H, m), 8. 44 (1H, d), 7. 24-7. 10 (5H, m), 7. 09 (1H, d), 5. 13 (1H, m), 4. 48 (1H, m), 3. 08 (1H, dd), 2. 78 (1H, dd), 2. 68 (1H, m), 1. 55 (2H, m), 1. 44 (1H, m), 0. 85 (3H, d), 0. 81 (3H, d), 0. 60 (2H, m), 0. 52 (2 H, m) | |
| | | | found, 441.2106 (-2. 6mmu) |
| 30 | 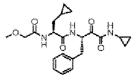 | DMSO-d6 | calcd for [M+H]⁺, 41 6.2180 |
| | | δ8. 71 (1H, d), 8. 39 (1H, d), 7. 52 (1H, d), 7. 30-7. 09 (5H, m), 5. 16 (1H, m), 4. 38 (1H, m), 3. 76 (2H, d), 3. 25 (3H, s), 3. 08 (1H, dd), 2. 75 (1H, dd), 2. 65 (1H, m), 1. 43 (2H, m), 0. 66-0. 47 (5H, m), 0. 30 (2H, m), 0. 00 (2H, m) | |
| | | | found, 416.2153 (-2. 7mmu) |
| 31 | 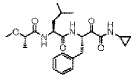 | DMSO-d6 | calcd for [M+H]⁺, 43 2.2493 |
| | | δ8. 71 (1H, d), 8. 33 (1H, d), 7. 60 (1H, d), 7. 28-7. 11 (5H, m), 5. 14 (1H, m), 4. 35 (1H, m), 3. 62 (1H, q), 3. 14 (3H, s), 3. 08 (1H, dd), 2. 74 (1H, dd), 2. 69 (1H, m), 1. 46 (1H, m), 1. 37 (2H, m), 1. 12 (3H, d), 0. 82 (3H, d), 0. 79 (3H, d), 0. 60 (2H, m), 0. 53 (2H, m) | |
| | | | found, 432.2466 (-2. 7mmu) |
| 32 | 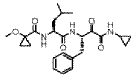 | DMSO-d6 | calcd for [M+H]⁺, 44 4.2493 |
| | | δ8. 71 (1H, d), 8. 31 (1H, d), 7. 69 (1H, d), 7. 29-7. 10 (5H, m), 5. 13 (1H, m), 4. 35 (1H, m), 3. 17 (3H, s), 3. 09 (1H, dd), 2. 75 (1H, dd), 2. 67 (1H, m), 1. 47 (1H, m), 1. 37 (2H, m), 0. 96 (4H, d), 0. 82 (3H, d), 0. 79 (3H, d), 0. 61 (2H, m), 0. 53 (2H, m) | |
| | | | found, 444.2483 (-1. 0mmu) |
| 33 | 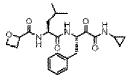 | DMSO-d6 | calcd for [M+H]⁺, 43 0. 2336 |
| | | δ8. 71 (1H, d), 8. 40 (1H, d), 7. 75 (1H, d), 7. 30-7. 10 (5H, m), 5. 13 (1H, m), 4. 85 (1H, m), 4. 61-4. 30 (3H, m), 3. 10 (1H, dd), 2. 88-2. 70 (3H, dm), 1. 61-1. 36 (3H, m), 0. 90-0. 74 (7H, m), 0. **61** (2H, m), 0. 54 (2H, m) | found, 430. 2333 (-0. 3mmu) |
| 34 | 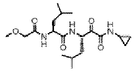 | DMSO-d6 | calcd for [M+H]⁺, 38 4. 2493 |
| | | δ8. 68 (1H, d), 8. 28 (1H, d), 7. 55 (1H, d), 4. 98 (1H, m), 4. 40 (1H, q), 3. 78 (2 H, s), 3. 26 (3H, s), 2. 70 (1H, m), 1. 64 (1H, m), 1. 55-1. 30 (5H, m), 0. 90-0. 80 (12H, m), 0. 61 (2H, m), 0. 53 (2H, m) | found, 384.2446 (-4. 7mmu) |
| 35 | 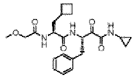 | DMSO-d6 | calcd for [M+H]⁺, 43 |
| | | δ8. 72 (1H, d), 8. 38 (1H, d), 7. 42 (1H, d), 7. 28-7. 12 (5H, m), 5. 13 (1H, m), 4. 24 (1H, m), 3. 74 (2H, d), 3. 24 (3H, s), 3. 08 (1H, dd), 2. 74 (1H, dd), 2. 70 (1H, m), 2. 15 (1H, m), 1. 90 (1H, m), 1. 90 (1H, m), 1. 75-1. 48 (6H, m), 0. 62 (2H, m)), 0. 54 (2H, m) | 0.2336 found, 430.2336 (0. 0mmu) |
| 36 | 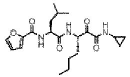 | DMSO-d6 | calcd for [M+H]⁺, 40 6. 2336 |
| | | δ8. 68 (1H, d), 8. 29 (1H, d), 8. 15 (1H, d), 7. 80 (1H, s), 7. 15 (1H, d), 6. 58 (1 H, dd), 4. 90 (1H, m), 4. 50 (1H, m), 2. 69 (1H, m), 1. 70 (1H, m), 1. 57 (2H, m), 1. 45 (2H, m), 1. 37-1. 14 (4H, m), 0. 92-0. 75 (9H, m), 0. 60 (2H, m), 0. 52 (2 H, m) | |
| | | | found, 406. 2304 (-3. 2mmu) |

**[Table 1D]**

| | | | |
|---|---|---|---|
| 37 | 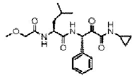 | | calcd for [M+H]⁺, 40 4. 2180 |
| | | | found, 404. 2169 (-1. 1mmu) |
| 38 | 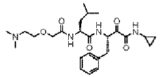 | | calcd for [M+H]⁺, 47 5. 2915 |
| | | | found, 475. 2906 (-0. 9mmu) |
| 39 | 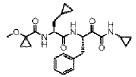 | | calcd for [M+H]⁺ , 44 2. 2336 |
| | | | found, 442. 2293 (-4. 3mmu) |
| 40 | 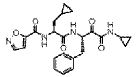 | | calcd for [M+H]⁺, 43 9. 1976 |
| | | | found, 439. 1945 (-3. 1mmu) |
| 41 | 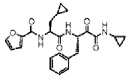 | DMSO-d6 | calcd for [M+H]⁺ , 43 8. 2023 |
| | | δ8. 68 (1H, d), 8. 34 (1H, d), 8. 09 (1H, d), 7. 81 (1H, d), 7. 27-7. 08 (6H, m), 6. 58 (1H, dd), 5. 16 (1H, m), 4. 47 (1H, m), 3. 07 (1H, dd), 2. 77 (1H, dd), 2. 6 7 (1H,m), 1. 58 (1H,m), 1. 45 (1H, m), 0. 72-0. 43 (5H, m), 0. 31 (2H, m), 0. 05 (2H, m) | |
| | | | found, 438. 1993 (-3. 0mmu) |
| 42 | 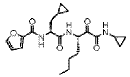 | DMSO-d6 | calcd for [M+H]⁺, 40 4. 2180 |
| | | δ8. 66 (1H, d), 8. 28 (1H, d), 8. 13 (1H, d), 7. 80 (1H, s), 7. 14 (1H, d), 6. 58 (1 H, dd), 4. 93 (1H, m), 4. 52 (1H, m), 2. 65 (1H, m), 1. 66 (2H, m), 1. 47 (2H, m), 1. 25 (4H, m), 0. 82 (3H, t), 0. 71 (1H, m), 0. 60 (2H, m), 0. 52 (2H, m), 0. 33 (2 H, m), 0. 06 (2H, m) | |
| | | | found, 404. 2151 (-2. 9mmu) |
| 43 | 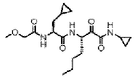 | DMSO-d6 | calcd for [M+H]⁺, 38 2. 2336 |
| | | δ8. 67 (1H, d), 8. 30 (1H, d), 7. 57 (1H, d), 4. 92 (1H, m), 4. 43 (1H, m), 3. 79 (2 H, s), 3. 28 (3H, s), 2. 70 (1H, m), 1. 69 (1H, m), 1. 50 (1H, m), 1. 45 (2H, m), 1. 36-1. 15 (5H, m), 0. 82 (3H, t), 0. 61 (2H, m), 0. 53 (2H, m), 0. 33 (2H, m), 0. 02 (2H, m) | |
| | | | found, 382. 2310 (-2. 6mmu) |
| 44 | 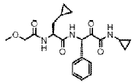 | | |

### <<Calpain Inhibitory Activity Measurement 1>>

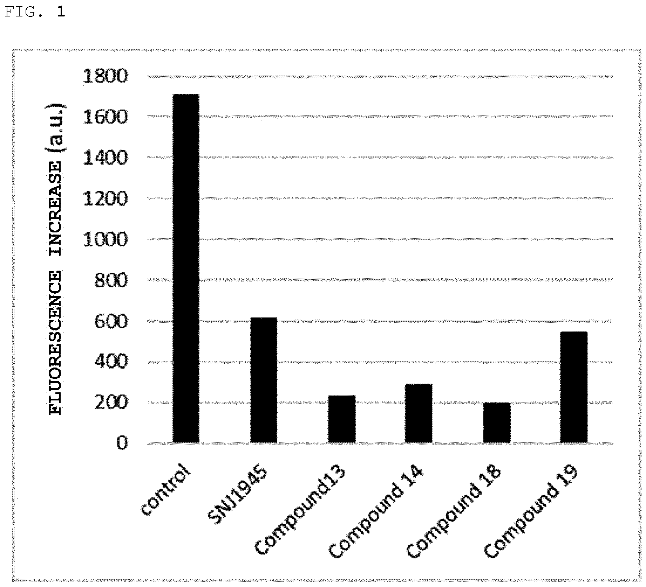

Calpain inhibitory activity was measured with SensorLyte^{™} 520 Calpain Activity Asay Kit (AnaSpec, Inc., AS-72149) in conformity with a method described in its assay protocol. SNJ-1945 (Medkoo Biosciences, Inc., Cat #564979), Compound 13, Compound 14, Compound 18, or Compound 19 serving as a calpain inhibitor and a calpain (calpain-1) in the kit were mixed with each other in Assay Buffer, and the mixture was incubated at 37°C for 3 minutes. 5-FAM/QXL^{™} 520 serving as a calpain enzyme substrate was added to the incubated product to perform an enzyme reaction by the calpain at 37°C for 25 minutes (the final concentration of each inhibitor was 0.062 µM, and the final concentration of 5-FAM/QXL^{™} 520 was 5.0 µM). In the enzyme reaction, the enzyme substrate is cleaved by the calpain to emit fluorescence. The emission fluorescence intensity of the substrate was detected with a plate reader (Infinite^{™} 200 PRO, TECAN) (at an excitation wavelength of 490 nm and a fluorescence wavelength of 520 nm). A fluorescence increase amount is shown in FIG. 1. SNJ-1945 is a known calpain inhibitor, and has the following structure.

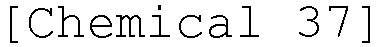

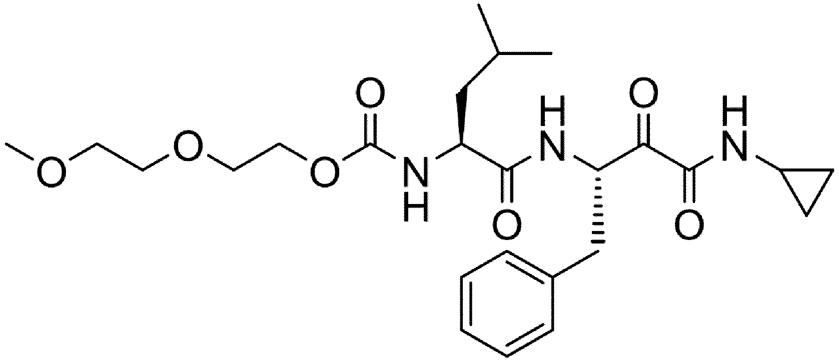

It is found from FIG. 1 that the enzyme activity of the calpain is inhibited by SNJ-1945. In each of Compound 13, Compound 14, and Compound 18, enzyme inhibitory activity stronger than that of SNJ-1945 was recognized. Meanwhile, the inhibitory activity of Compound 19 was comparable to that of SNJ-1945. It was suggested from the foregoing that a calpain inhibitory ability was improved by bonding an amide group having α-carbon, which was bonded to an oxygen atom or a sulfur atom, to the N-terminal of the compound represented by the formula (I).

### <<Calpain Inhibitory Activity Measurement 2>>

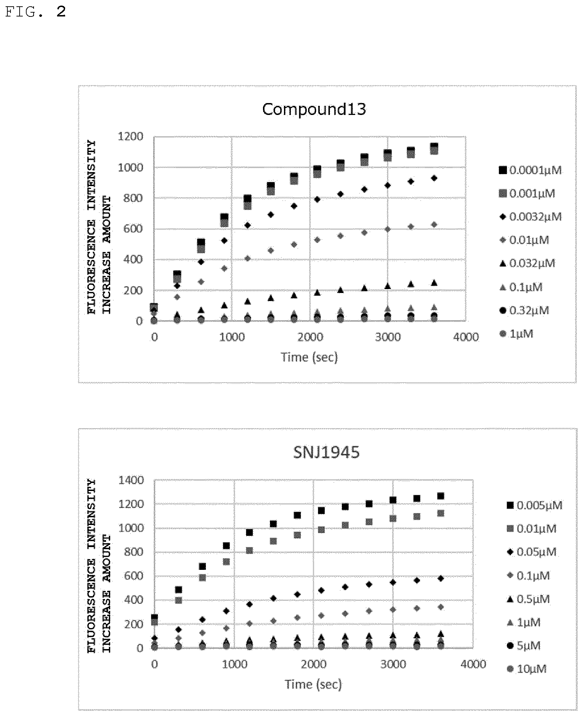

Calpain-1 (calpain-1 from human erythrocytes, Cat. No. 208713, Merck Millipore) and a DMSO solution of a HMRG fluorescent probe for detecting calpain activity were added to each of 50 mM Tris buffers (pH: 7.4, 5 mM CaCl₂, 2 mM DTE, 2 mM EGTA) containing various concentrations of SNJ-1945 or Compound 13 to perform fluorescence assays (the final concentration of the fluorescent probe was 5 µM, and the final concentration of calpain-1 was 6.48 µg/mL). An enzyme reaction was performed at 37°C, and the fluorescence spectrum of the reaction solution was observed with time for 30 minutes from the start of the reaction (at an excitation wavelength of 460 nm and a fluorescence wavelength of 530 nm). The results are shown in FIG. 2 (concentrations shown in the figure each represent the final concentration of SNJ-1945 or Compound 13 in each assay). The following compound was used as the probe for detecting calpain activity.

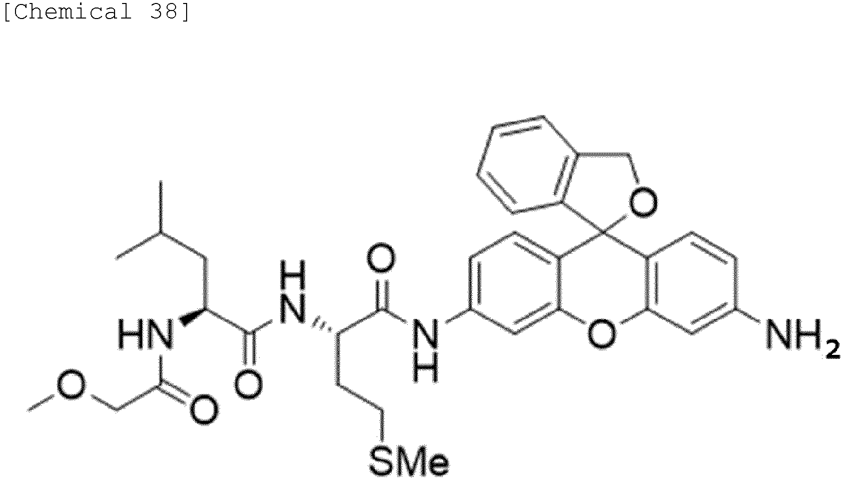

An IC₅₀ was determined on the basis of a graph produced with data analysis software "KaleidaGraph". As a result, the IC₅₀ of Compound 13 was 0.010 µM, and the IC₅₀ of SNJ-1945 was 0.040 µM.

### <<Calpain Inhibitory Activity Measurement 3>>

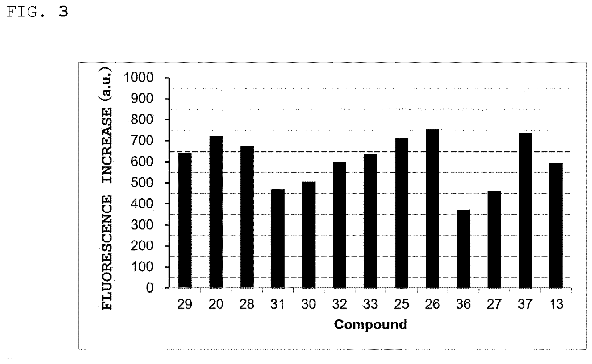

Calpain-1 (calpain-1 from human erythrocytes, Cat. No. 208713, Merck Millipore) and a DMSO solution of a HMRG fluorescent probe for detecting calpain activity were added to a 50 mM Tris buffer (pH: 7.4, 5 mM CaCl₂, 2 mM DTE, 2 mM EGTA) containing Compound 13, 20, 25, 26, 27, 28, 29, 30, 31, 32, 33, 36, or 37 to perform fluorescence assays (the final concentration of each compound was 0.062 µM, the final concentration of the fluorescent probe was 5 µM, and the final concentration of calpain-1 was 6.48 µg/mL). An enzyme reaction was performed at 37°C, and the fluorescence spectrum of the reaction solution was observed with time for 30 minutes from the start of the reaction (at an excitation wavelength of 460 nm and a fluorescence wavelength of 530 nm). The results are shown in FIG. 3. The same compound as that of the above-mentioned measurement 2 was used as the probe for detecting calpain activity.

As shown in FIG. 3, each of the compounds showed calpain inhibitory activity comparable to or more than that of Compound 13.

### Industrial Applicability

The compound (I), calpain inhibitor, or pharmaceutical composition of the present invention may be suitably used in the treatment or prevention of a calpain activity-associated disease.

## Claims

1. A compound represented by the following general formula (I) or a salt thereof: 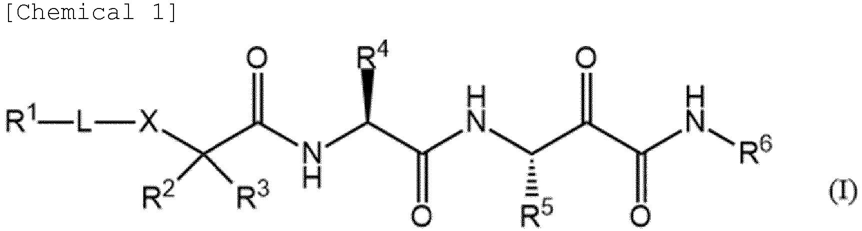 in the formula (I),
R¹ represents a hydrogen atom, a linear, branched, or cyclic alkyl group that may be substituted, a linear or branched alkenyl group that may be substituted, a linear or branched alkynyl group that may be substituted, an aryl group that may be substituted, or a heterocyclic group that may be substituted, or may be bonded to R² to form a ring,
R² and R³ each independently represent a hydrogen atom, a hydroxyl group, an alkoxy group that may be substituted, or a linear, branched, or cyclic alkyl group that may be substituted, or R² and R³ may be bonded to form a ring, provided that when R² is bonded to R¹ to form a double bond-containing ring, R³ may be absent,
R⁴ represents a hydrogen atom, or a linear or branched alkyl group having 4 or more carbon atoms that may be substituted,
R⁵ represents a hydrogen atom, a linear or branched alkyl group that may be substituted, a linear or branched alkenyl group that may be substituted, a linear or branched alkynyl group that may be substituted, an aryl group that may be substituted, or a heterocyclic group that may be substituted,
R⁶ represents a linear, branched, or cyclic alkyl group that may be substituted, a linear or branched alkenyl group that may be substituted, a linear or branched alkynyl group that may be substituted, an aryl group that may be substituted, a heterocyclic group that may be substituted, a fused polycyclic hydrocarbon group that may be substituted, an alkoxy group that may be substituted, or a hydrogen atom,
X represents O or S,
when R¹ and R² are bonded to form a ring, R¹ may represent O, S, or N, and R² may represent O or S, and
L represents an amide bond, an ester bond, -NH-, -N-(linear, branched, or cyclic alkylene)-, or -(linear or branched alkylene-O-)ₙ- where "n" represents an integer of from 1 to 10, or L represents a single bond.

2. The compound or the salt thereof according to claim 1, wherein in the formula (I), a group represented by -X-L-R¹ is a group represented by the following formula (II): 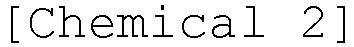 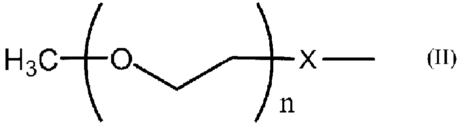 in the formula (II), "n" represents an integer of from 0 to 10.

3. The compound or the salt thereof according to claim 1, wherein in the formula (I), a group represented by -X-L-R¹ is a hydroxyl group, a thiol group, or a group selected from the following.

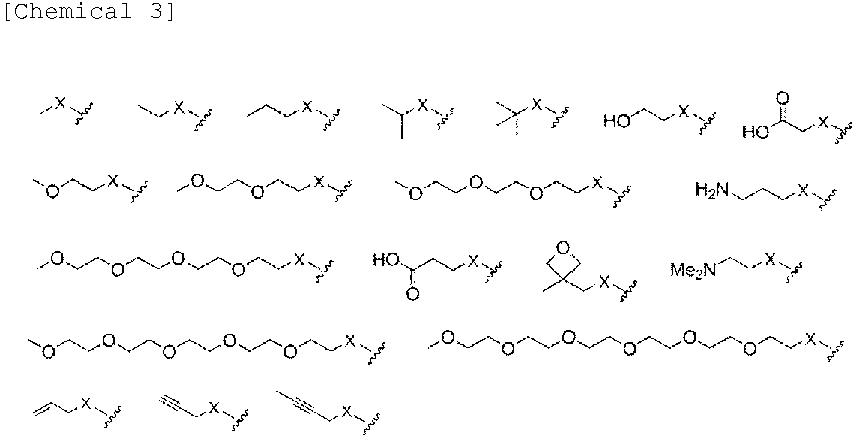

4. The compound or the salt thereof according to claim 1, wherein in the formula (I), a partial structure represented by 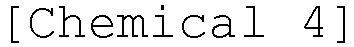 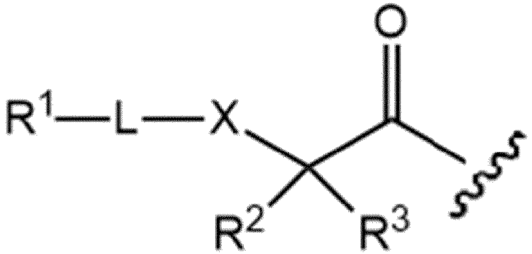 is selected from structures represented by the following formulae: 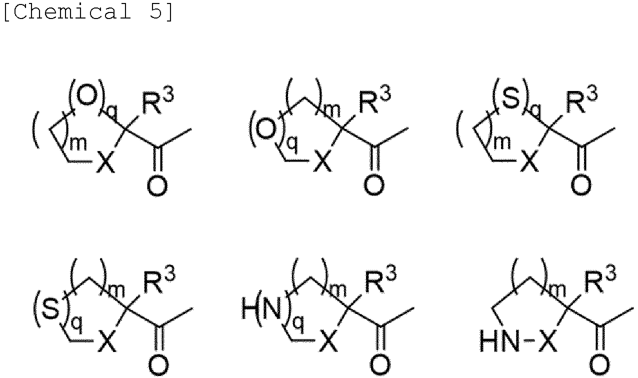 where "m" represents an integer of from 1 to 3, and "q" represents 0 or 1, and structures represented by the following formulae: 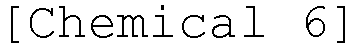 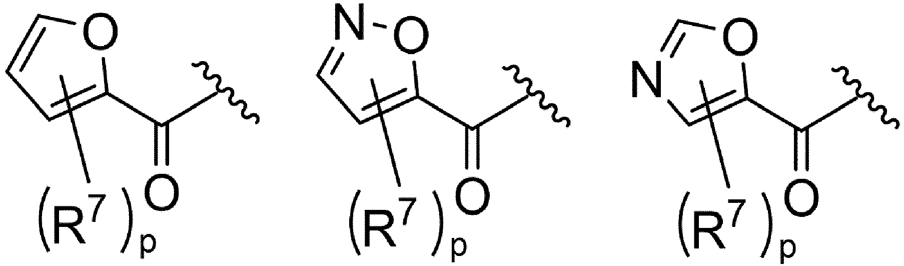 where R⁷s may be identical to or different from each other, and each represent a substituent bonded to a heterocycle, and "p" represents an integer of from 0 to 3.

5. The compound or the salt thereof according to claim 1, wherein R² and R³ each independently represent a hydrogen atom or an alkyl group, or are bonded to each other to form a cycloalkyl group.

6. The compound or the salt thereof according to claim 1, wherein in the formula (I), R⁴ represents an isobutyl group, a sec-butyl group, a cyclopropylmethyl group, or a cyclobutylmethyl group.

7. The compound or the salt thereof according to claim 1, wherein in the formula (I), R⁵ represents a benzyl group, a phenylethyl group, a 2-(methylthio)ethyl group, a 2-(methylsulfinyl)ethyl group, a n-butyl group, a 1-hydroxyethyl group, a 3-guanidinopropyl group, a 4-aminobutyl group, a 1H-imidazol-4-yl-methyl group, a phenyl group, a n-propyl group, or an isobutyl group.

8. The compound or the salt thereof according to claim 1, wherein the compound is selected from compounds represented by the following formulae: 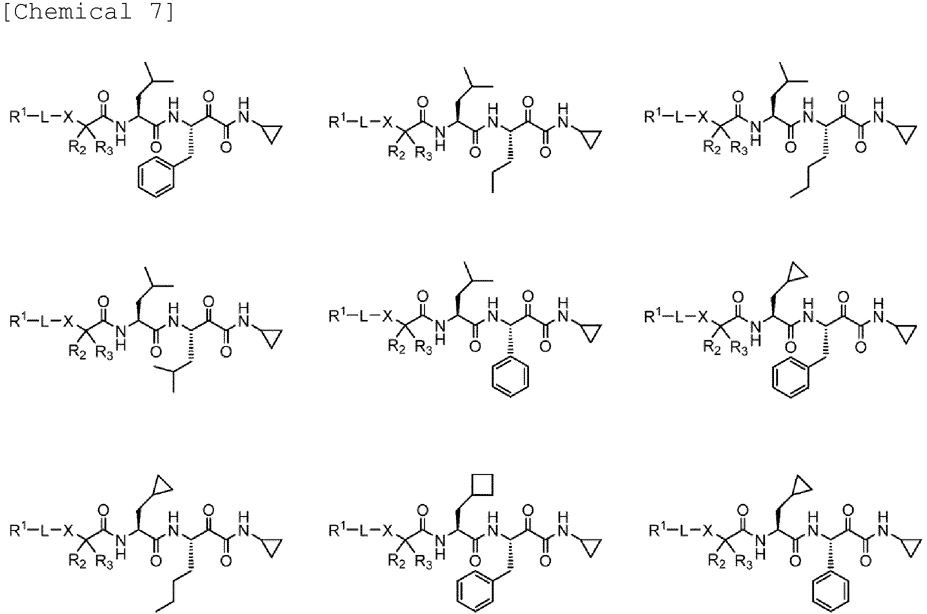 where R¹, R², and R³ are each as defined for the formula (I).

9. The compound or the salt thereof according to claim 1, wherein the compound is selected from compounds represented by the following formulae: 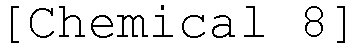 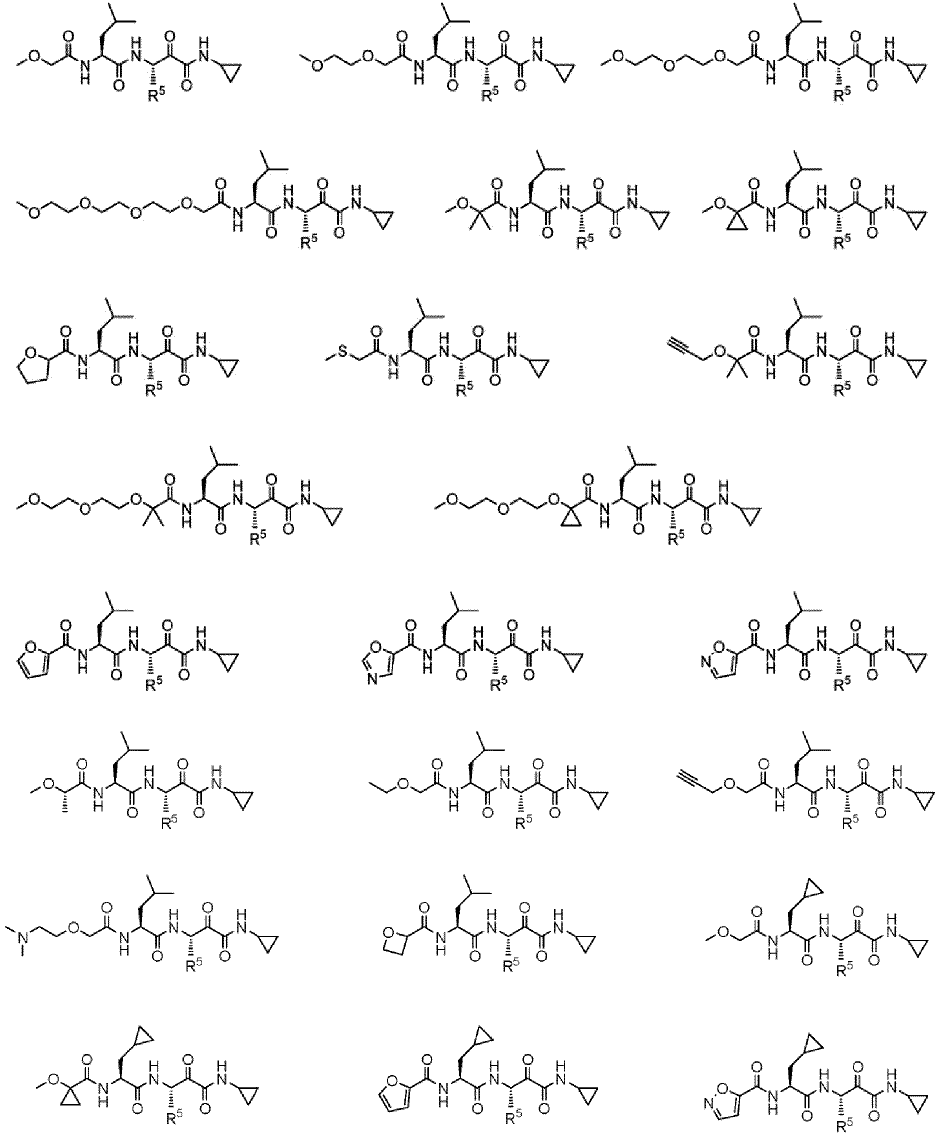 where R⁵ is as defined for the formula (I).

10. The compound or the salt thereof according to claim 9, wherein R⁵ represents a benzyl group, a phenylethyl group, a 2-(methylthio)ethyl group, a 2-(methylsulfinyl)ethyl group, a n-butyl group, a 1-hydroxyethyl group, a 3-guanidinopropyl group, a 4-aminobutyl group, a 1H-imidazol-4-yl-methyl group, a phenyl group, a n-propyl group, or an isobutyl group.

11. A calpain inhibitor, comprising the compound or the salt thereof of any one of claims 1 to 10.

12. A pharmaceutical composition for treating or preventing a calpain activity-associated disease, comprising the compound or the salt thereof of any one of claims 1 to 10.

13. The pharmaceutical composition according to claim 12, wherein the calpain activity-associated disease is an eye disease, a muscle disease, diabetes, an inflammatory disease, an autoimmune disease, a neurological disease, a heart or blood vessel disease, a cancer, a brain tumor, an aging syndrome, progeria, an infectious disease, traumatic encephalopathy, Machado-Joseph disease, preeclampsia, or pulmonary fibrosis.

14. The pharmaceutical composition according to claim 13, wherein the eye disease is glaucoma, autosomal dominant neovascular inflammatory vitreoretinopathy, retinitis pigmentosa, age-related macular degeneration, retinal neuropathy or a retinal vascular occlusive disease associated with diabetes, retinal ischemia, or cataract.

15. The pharmaceutical composition according to claim 14, wherein the glaucoma is normal-tension glaucoma.

16. A method of treating or preventing a calpain activity-associated disease, comprising administering an effective dose of the compound or the salt thereof of any one of claims 1 to 10 to an individual in need of the treatment or prevention of the calpain activity-associated disease.
